# EUROPEAN PATENT APPLICATION

(11) **EP 3 705 119 A1**
(43) Date of publication of application: **09.09.2020**
(21) Application number: 18873084.0
(22) Date of filing: 30.10.2018
(51) Int. Cl.: A61K 31/045, A61K 9/107, A61K 47/12, A61K 47/14, A61K 47/26, A61K 47/44, A61P 1/00

(54) **PHARMACEUTICAL COMPOSITION, METHOD FOR STABILISNG PHARMACEUTICAL COMPOSITION, AND METHOD FOR EVALUATING STORAGE STABILITY OF PHARMACEUTICAL COMPOSITION**

(30) Priority: 01.11.2017 JP 2017212266; 27.11.2017 JP 2017227259
(71) Applicant: Nihon Pharmaceutical Co., Ltd., Tokyo 104-0044 (JP)
(72) Inventor: HAMAWAKI, Tomonori, Narita-shi Chiba 286-0825 (JP); OIZUMI, Kumiko, Narita-shi Chiba 286-0825 (JP); IWASAKI, Noriyuki, Narita-shi Chiba 286-0825 (JP)
(74) Representative: Grünecker Patent- und Rechtsanwälte PartG mbB
(86) International application number: PCT/JP2018/040921
(87) International publication number: WO 2019/088274

(57) **Abstract**

Provided is a pharmaceutical composition having excellent storage stability even when containing 1-menthol in a high concentration. The pharmaceutical composition has a cloud point of 30°C to 75°C and contains components (A), (B), (C), (D), and (E) as follows:
Component (A): 1-menthol;
Component (B): at least one selected from the group consisting of a medium-chain fatty acid triacylglycerol, a medium-chain fatty acid, and a salt of a medium-chain fatty acid;
Component (C): a nonionic surfactant having a hydrophile-lipophile balance (HLB) of not less than 10;
Component (D): a polyhydric alcohol having not less than three hydroxy groups per molecule and/or a poly(oxyalkylene) glycol; and
Component (E): water.

## Description

### Technical Field

The present invention relates to a pharmaceutical composition, a method for stabilizing a pharmaceutical composition, and a method for evaluating the storage stability of a pharmaceutical composition. This application claims priority to Japanese Patent Application No. 2017-212266 filed in Japan November 1, 2017; and to Japanese Patent Application No. 2017-227259 filed in Japan November 27, 2017, the entire contents of which applications are incorporated herein by reference.

### Background Art

The use of pharmaceutical compositions containing 1-menthol (hereinafter also referred to as "preparation(s)") as gastrointestinal smooth muscle contraction inhibitors have been examined. 1-Menthol is almost insoluble in water. As a possible solution to this, an exemplary disclosed technique emulsifies such an 1-menthol-containing composition using a sorbitan mono-fatty acid ester, which works as a surfactant (see Patent Literature (PTL) 1).

Another known technique regulates 1-menthol in an emulsion to have an average particle size of less than 100 nm (see PTL 2), and still another known technique combines a 1-menthol-containing composition with benzoic acids and a surfactant such as a polyoxyethylene hydrogenated castor oil or a sucrose fatty acid ester (see PTL 3). These techniques give gastrointestinal smooth muscle contraction inhibitors that are stable and highly transparent.

### Citation List

### Patent Literature

PTL 1: Japanese Unexamined Patent Application Publication (JP-A) No. 2003-292450
PTL 2: PCT International Publication Number WO2003/097026
PTL 3: JP-A No. 2005-179292

### Summary of Invention

### Technical Problem

The pharmaceutical compositions are usable also for patients with pre-existing diseases such as glaucoma, prostatic hyperplasia, and arrhythmia, are highly useful, and are widely used for restraining or inhibiting peristaltic movement (peristalsis; contraction) during gastroscopic examination and/or gastroscopic treatment (hereinafter also referred to as "upon gastroscopy").

Demands are made to provide pharmaceutical compositions having high storage stability even though containing 1-menthol in a high concentration (for example, higher than 0.8 weight %).

The present invention has an object to provide a pharmaceutical composition that is highly stable during storage even when containing 1-menthol in a high concentration.

The present invention has another object to provide a method for stabilizing a pharmaceutical composition, which method allows the pharmaceutical composition to have excellent emulsion stability (stability as an emulsion).

The present invention has still another object to provide a method for evaluating the storage stability of a pharmaceutical composition, which method enables designing of a pharmaceutical composition that is highly stable with time.

In addition, the present invention has still another object to provide a gastrointestinal smooth muscle contraction inhibitor that is highly stable at low temperatures even when containing 1-menthol in a high concentration.

### Solution to Problem

After intensive investigations to achieve the objects, the inventors of the present invention have found that a specific pharmaceutical composition has excellent storage stability. This pharmaceutical composition has a cloud point of 30°C to 75°C, and contains the components of: (A) 1-menthol; (B) at least one selected from the group consisting of a medium-chain fatty acid triacylglycerol, a medium-chain fatty acid, and a salt of medium-chain fatty acid; (C) a nonionic surfactant having a hydrophile-lipophile balance (HLB) of not less than 10; (D) a polyhydric alcohol having not less than three hydroxy groups per molecule and/or a poly(oxyalkylene) glycol; and (E) water.

Also after intensive investigations to achieve the objects, the inventors have found that a specific gastrointestinal smooth muscle contraction inhibitor is highly stable at low temperatures. This gastrointestinal smooth muscle contraction inhibitor contains the components of (A) 1-menthol; (b1) a medium-chain fatty acid triacylglycerol; (b2) a medium-chain fatty acid and/or a salt thereof; (F) at least one selected from the group consisting of sucrose fatty acid esters, polyoxyethylene hydrogenated castor oils, and polysorbates; and (G) at least one selected from the group consisting of benzoic acid, p-hydroxybenzoic acid, and a salt or an alkyl ester thereof. The inhibitor contains the components (A), (b1), (b2), (F), and (G) in contents or proportions within specific ranges. The inhibitor has the weight ratio of the content of component (b2) to that of the component (G) within a specific range. The present invention has been made on the basis of these findings.

Specifically, the present invention provides, in an embodiment, a pharmaceutical composition containing components (A), (B), (C), (D), and (E) as follows and having a cloud point of 30°C to 75°C. This pharmaceutical composition is hereinafter also referred to as a "pharmaceutical compositions according to the first embodiment". The components (A), (B), (C), (D), and (E) are as follows:
Component (A): 1-menthol;
Component (B): at least one selected from the group consisting of a medium-chain fatty acid triacylglycerol, a medium-chain fatty acid, and a salt of medium-chain fatty acid;
Component (C): a nonionic surfactant having an HLB of not less than 10;
Component (D): a polyhydric alcohol having not less than three hydroxy groups per molecule and/or a poly(oxyalkylene) glycol; and
Component (E): water.

The weight ratio of the content of the component (C) to that of the component (D) [component (C) / component (D)] is preferably 0.2 to 5.0.

The component (C) is preferably at least one selected from the group consisting of a sucrose fatty acid ester, a polyoxyethylene hydrogenated castor oil, and a polysorbate.

The component (C) preferably has a content of the sucrose fatty acid ester of not more than 0.1 weight %.

The component (D) is preferably at least one selected from the group consisting of glycerol, D-sorbitol, D-mannitol, and macrogol 400.

The pharmaceutical composition preferably has a pH of 3 to 8.

The content of component (A) is preferably 0.01 to 5 weight %. The content of component (B) is preferably 0.1 to 10 weight %. The content of component (C) is preferably 3 to 15 weight %. The content of component (D) is preferably present in a content of 3 to 20 weight %.

Preferably, the pharmaceutical composition is an emulsion, and particles in the emulsion have an average particle size (average size of particles) of less than 100 nm.

The pharmaceutical composition preferably has a light transmittance of not less than 50%.

The pharmaceutical composition is preferably a gastrointestinal smooth muscle contraction inhibitor.

The present invention also provides, in another aspect, a method for inhibiting gastrointestinal smooth muscle contraction. The method includes administering to a mammal the pharmaceutical composition.

The present invention also provides, in still another aspect, use of the pharmaceutical composition for inhibiting gastrointestinal smooth muscle contraction.

The present invention also provides, in still another aspect, a method for stabilizing a pharmaceutical composition. The pharmaceutical composition includes 1-menthol, at least one selected from the group consisting of a medium-chain fatty acid triacylglycerol, a medium-chain fatty acid, and a salt of medium-chain fatty acid, a nonionic surfactant having a hydrophile-lipophile balance (HLB) of not less than 10, and water. The method includes combining the pharmaceutical composition with a polyhydric alcohol having not less than three hydroxy groups per molecule and/or a poly(oxyalkylene) glycol, adjusting the content each of the components, making the cloud point of the pharmaceutical composition 30°C to 75°C, and whereby stabilizing the emulsified state of the pharmaceutical composition.

The present invention provides, in still another aspect, a method for evaluating storage stability of a pharmaceutical composition containing components (A), (B), (C), and water. The method includes measuring a cloud point of the pharmaceutical composition, determining a difference between the cloud point and a storage temperature of the pharmaceutical composition, and evaluating the storage stability of the pharmaceutical composition by taking the difference as an index. The components (A), (B), and (C) are as follows:
Component (A): 1-menthol;
Component (B): at least one selected from the group consisting of a medium-chain fatty acid triacylglycerol, a medium-chain fatty acid, and a salt of medium-chain fatty acid; and
Component (C): a nonionic surfactant having an HLB of not less than 10.

The present invention provides, in another embodiment, a gastrointestinal smooth muscle contraction inhibitor. The gastrointestinal smooth muscle contraction inhibitor contains components (A), (b1), (b2), (F), and (G). The content of component (A) is 0.01 to 5 weight %. The content of component (b1) is 0.1 to 10 times as much by weight as that of the component (A). The content of component (b2) is 0.1 to 10 times as much by weight as that of the component (A). The content of component (F) is 0.1 to 10 times as much by weight as the total amount of the component (A), the component (b1), and the component (b2). The content of component (G) is 0.01 to 2.0 weight % as benzoic acid. The weight ratio of content of the component (b2) to that of the component (G) [component (b2) / component (G)] is from 1/5 to 10/1. The components (A), (b1), (b2), (F), and (G) are as follows:
Component (A): 1-menthol;
Component (b1): a medium-chain fatty acid triacylglycerol;
Component (b2): a medium-chain fatty acid and/or a salt thereof;
Component (F): at least one selected from the group consisting of a sucrose fatty acid ester, a polyoxyethylene hydrogenated castor oil, and a polysorbate; and
Component (G): at least one selected from the group consisting of benzoic acid, p-hydroxybenzoic acid, and a salt or an alkyl ester thereof.

This gastrointestinal smooth muscle contraction inhibitor is hereinafter also referred to as a "gastrointestinal smooth muscle contraction inhibitor according to the second embodiment".

The content of component (A) is preferably more than 0.8 weight % but not more than 3 weight %.

The gastrointestinal smooth muscle contraction inhibitor preferably includes, as the component (F), a sucrose fatty acid ester, a polyoxyethylene hydrogenated castor oil, and a polysorbate.

The gastrointestinal smooth muscle contraction inhibitor preferably includes a sucrose fatty acid ester as the component (F), and the sucrose fatty acid ester preferably includes a sucrose fatty acid monoester in a proportion of not less than 55 weight % of the total amount of the sucrose fatty acid ester.

The component (b1) is preferably a triacylglycerol of a C₆-C₁₂ fatty acid.

The component (b2) is preferably a C₆-C₁₂ fatty acid and/or a salt thereof.

Preferably, the smooth muscle contraction inhibitor is an emulsion, and particles in the emulsion have an average particle size of less than 100 nm.

The gastrointestinal smooth muscle contraction inhibitor preferably has a light transmittance of not less than 50%.

The present invention also provides, in another aspect, a method for inhibiting gastrointestinal smooth muscle contraction. The method includes administering to a mammal a pharmaceutical composition. The pharmaceutical composition contains components (A), (b1), (b2), (F), and (G) as follows. The content of component (A) is 0.01 to 5 weight %. The content of component (b1) is 0.1 to 10 times as much by weight as that of the component (A). The content of component (b2) is 0.1 to 10 times as much by weight as that of the component (A). The content of component (F) is 0.1 to 10 times as much by weight as the total amount of the component (A), the component (b1), and the component (b2). The content of component (G) is 0.01 to 2.0 weight % as benzoic acid. The weight ratio of the component (b2) to the component (G) [component (b2) / component (G)] is from 1/5 to 10/1. The components (A), (b1), (b2), (F), and (G) are as follows:
Component (A): 1-menthol;
Component (b1): a medium-chain fatty acid triacylglycerol;
Component (b2): a medium-chain fatty acid and/or a salt thereof;
Component (F): at least one selected from the group consisting of a sucrose fatty acid ester, a polyoxyethylene hydrogenated castor oil, and a polysorbate; and
Component (G): at least one selected from the group consisting of benzoic acid, p-hydroxybenzoic acid, and a salt or an alkyl ester thereof.

The present invention provides, in still another aspect, use of a pharmaceutical composition for inhibiting gastrointestinal smooth muscle contraction. The pharmaceutical composition contains components (A), (b1), (b2), (F), and (G). The content of component (A) is 0.01 to 5 weight %. The content of component (b1) is 0.1 to 10 times as much by weight as that of the component (A). The content of component (b2) is 0.1 to 10 times as much by weight as that of the component (A). The content of component (F) is 0.1 to 10 times as much by weight as the total amount of the component (A), the component (b1), and the component (b2). The content of component (G) is 0.01 to 2.0 weight % as benzoic acid. The weight ratio of the component (b2) to the component (G) [component (b2) / component (G)] is from 1/5 to 10/1. The components (A), (b1), (b2), (F), and (G) are as follows:
Component (A): 1-menthol;
Component (b1): a medium-chain fatty acid triacylglycerol;
Component (b2): a medium-chain fatty acid and/or a salt thereof;
Component (F): at least one selected from the group consisting of a sucrose fatty acid ester, a polyoxyethylene hydrogenated castor oil, and a polysorbate; and
Component (G): at least one selected from the group consisting of benzoic acid, p-hydroxybenzoic acid, and a salt or an alkyl ester thereof.

As used herein, the term "XX to YY" is used to mean that both the numerical values XX and YY are included respectively as the lower limit and the upper limit.

Also as used herein, the term "weight %" refers to a ratio relative to the total amount (100 weight %) of the pharmaceutical composition according to the first embodiment of the present invention or the gastrointestinal smooth muscle contraction inhibitor according to the second embodiment of the present invention, unless otherwise specified.

### Advantageous Effects of Invention

The pharmaceutical composition according to the first embodiment of the present invention is highly stable during storage, even when containing 1-menthol in a high concentration. The method according to the first embodiment of the present invention for stabilizing a pharmaceutical composition can give a preparation that is highly stable when existing as an emulsion. The method according to the first embodiment of the present invention for evaluating the storage stability of a pharmaceutical composition enables easy designing of a pharmaceutical composition that is highly stable with time.

The gastrointestinal smooth muscle contraction inhibitor according to the second embodiment of the present invention is highly stable at low temperatures, even when containing 1-menthol in a high concentration.

### Brief Description of Drawing

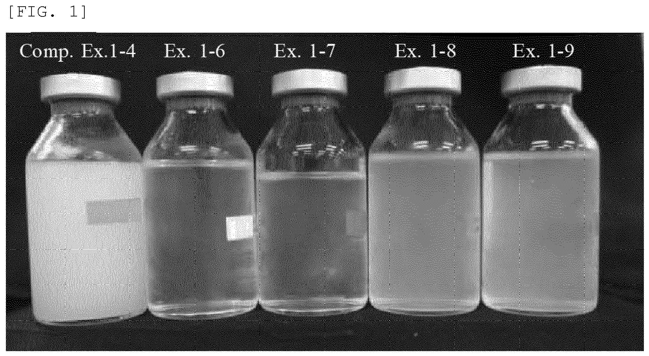

FIG. 1 is a photograph illustrating the appearances of pharmaceutical compositions according to Examples 1-6 to 1-9 (Ex. 1-6 to 1-9) and Comparative Example 1-4 (Comp. Ex. 1-4) regarding the first embodiment of the present invention, after storage at 4°C for one month.

### Description of Embodiments

### First Embodiment

A pharmaceutical composition according to the first embodiment of the present invention is preferably used as a gastrointestinal smooth muscle contraction inhibitor at gastroscopic, colonoscopic, or another gastrointestinal endoscopic examination and/or treatment (hereinafter also referred to as "upon gastrointestinal endoscopy"). The pharmaceutical composition according to the first embodiment of the present invention includes 1-menthol; at least one selected from the group consisting of a medium-chain fatty acid triacylglycerol, a medium-chain fatty acid, and a salt of medium-chain fatty acid; a nonionic surfactant having an HLB of not less than 10; a polyhydric alcohol having not less than three hydroxy groups per molecule and/or a poly(oxyalkylene) glycol; and water.

The pharmaceutical composition according to the first embodiment of the present invention has a cloud point of 30°C to 75°C, more preferably 35°C to 70°C, and still more preferably 35°C to 65°C. Control of the cloud point within the temperature range allows the pharmaceutical composition to have excellent emulsion stability and storage stability in particular during storage at low temperatures.

The present invention provides, as another aspect of the first embodiment of the present invention, a method for inhibiting gastrointestinal smooth muscle contraction. This method includes administering to a mammal a specific pharmaceutical composition. This pharmaceutical composition has a cloud point of 30°C to 75°C and includes 1-menthol; at least one selected from the group consisting of a medium-chain fatty acid triacylglycerol, a medium-chain fatty acid, and a salt of medium-chain fatty acid; a nonionic surfactant having an HLB of not less than 10; a polyhydric alcohol having not less than three hydroxy groups per molecule and/or a poly(oxyalkylene) glycol; and water.

The present invention also provides, as still another aspect of the first embodiment of the present invention, use of a specific pharmaceutical composition for inhibiting gastrointestinal smooth muscle contraction. This pharmaceutical composition has a cloud point of 30°C to 75°C and includes 1-menthol; at least one selected from the group consisting of a medium-chain fatty acid triacylglycerol, a medium-chain fatty acid, and a salt of medium-chain fatty acid; a nonionic surfactant having an HLB of not less than 10; a polyhydric alcohol having not less than three hydroxy groups per molecule and/or a poly(oxyalkylene) glycol; and water.

As used herein, 1-menthol is also referred to as a "component (A)"; the at least one selected from the group consisting of a medium-chain fatty acid triacylglycerol, a medium-chain fatty acid, and a salt of medium-chain fatty acid is also referred to as a "component (B)"; the nonionic surfactant having an HLB of not less than 10 is also referred to as a "component (C)"; a polyhydric alcohol having not less than three hydroxy groups per molecule and/or a poly(oxyalkylene) glycol is also referred to as a "component (D)"; and the water is also referred to as a "component (E)".

Possible components to exist in the pharmaceutical composition according to the first embodiment of the present invention, namely, the component (A), the component (B), the component (C), the component (D), the component (E), and components other than the components (A) to (E) may be used alone or in combination in each category.

As used herein, the term "cloud point" refers to the temperature at which the pharmaceutical composition according to the first embodiment of the present invention becomes entirely cloudy and loses its transparency when the temperature of the pharmaceutical composition (preparation) is raised.

The cloud point is measured under conditions as follows. The pharmaceutical composition according to the first embodiment of the present invention is placed in an airtight container (for example, a cylindrical transparent container having a diameter of 20 to 40 mm, preferably a cylindrical transparent container having a diameter of 30 mm), gradually heats with stirring on a water bath, and how the preparation appears is visually observed. During visual observation, the temperature at which the entire preparation becomes cloudy and loses its transparency is measured as the cloud point. The heating is not limited in rate of temperature rise. The stirring may be performed under any conditions, but is preferably performed as stirring with high-speed revolution, to provide a homogeneous heat distribution and to give a homogeneous composition.

### Component (A): 1-Menthol

The component (A) is 1-menthol. It is considered that 1-menthol binds to a voltage-dependent L-type calcium channel on the cell membrane of a smooth muscle, and this blocks calcium ions from entering the cell, eliminates or minimizes the membrane potential occurrence, and relaxes the smooth muscle. When the pharmaceutical composition according to the first embodiment of the present invention sprays or is sprayed to the wall of a digestive tract (digestive organ), 1-menthol permeates through the mucosa and reaches, directly acts upon, and relaxes the smooth muscle to inhibit or restrain the contraction of the smooth muscle.

1-Menthol is a principal component of peppermint oil and is present therein in a content of not less than 30 weight %. The peppermint oil results from steam distillation of peppermint (*Mentha piperita*) or Japanese mint (*Mentha avensis*). Such a 1-menthol-containing material usable herein may be peppermint oil, or mint oil, or another similar oil, but is preferably one that has been highly purified from peppermint oil or another oil typically by fractional distillation. The material for use herein is still more preferably one having a purity of not less than 90 weight %. Such 1-menthol-containing materials are recently also available by synthesis. The 1-menthol for use in the present invention is preferably one that conforms to the specifications for 1-menthol as prescribed in Japanese Pharmacopoeia.

A non-limiting example of commercial products of the component (A) is 1-Menthol (trade name, from The Suzuki Menthol Co., Ltd.).

The pharmaceutical composition according to the first embodiment of the present invention contains the component (A) in a content of preferably 0.01 to 5 weight %, more preferably 0.1 to 4 weight %, still more preferably from more than 0.8 weight % but not more than 3 weight %, and most preferably 1 to 2 weight %, of the total amount (100 weight %) of the pharmaceutical composition. The pharmaceutical composition, when containing the component (A) in a content of not less than 0.01 weight %, can sufficiently relax the smooth muscles in a digestive organ. The pharmaceutical composition, when containing the component (A) in a content of not more than 5 weight %, is more stable over a long time approximately without precipitation even at low temperatures.

### Component (B): At least one selected from the group consisting of a medium-chain fatty acid triacylglycerol, a medium-chain fatty acid, and a salt of medium-chain fatty acid

The component (B) is at least one selected from the group consisting of a medium-chain fatty acid triacylglycerol, a medium-chain fatty acid, and a salt of medium-chain fatty acid. The component (B) advantageously dissolves or solubilizes the component (A) in a solvent. The pharmaceutical composition may contain each of different components (B) alone or in combination.

The medium-chain fatty acid triacylglycerols may be any ones usable as preparation additives, but are preferably triacylglycerols each derived from C₆-C₁₂ fatty acids alone or in combination as a mixture. This configuration is preferred for more excellent stability of the pharmaceutical composition. Non-limiting examples of the C₆-C₁₂ fatty acids include caproic acid (C6), caprylic acid (C8), capric acid (C10), and lauric acid (C12). Among them, tricaprylin (glycerol tricaprylate), which is derived from three moieties of a C8 fatty acid alone, is particularly preferred from the viewpoint of excellent stability.

The medium-chain fatty acids and the salts of medium-chain fatty acid may be any ones usable as preparation additives, but are preferably C₆-C₁₂ fatty acids and/or salts of these fatty acids, for excellent stability. Non-limiting examples of the C₆-C₁₂ fatty acids include caproic acid (C6), caprylic acid (C8), capric acid (C10), and lauric acid (C12). The salts of medium-chain fatty acid may be any pharmacologically acceptable salts, but are preferably alkali metal salts such as sodium salts and potassium salts. Among them, caprylic acid, which contains 8 carbon atoms, and/or an alkali metal salt of caprylic acid (such as sodium caprylate or potassium caprylate) is preferably employed.

The component (B) usable herein may be selected from commercial products. Non-limiting examples of the commercial products of the medium-chain fatty acid triacylglycerols include ones available under the trade names of: COCONARD RK, COCONARD MT, and COCONARD ML (each from Kao Corporation); and PANACET 800 (from NOF CORPORATION). Non-limiting examples of the commercial products of the medium-chain fatty acids include ones available under the trade names of: Hexanoic Acid (from Wako Pure Chemical Corporation); Caprylic acid (from Merck Millipore); Decanoic Acid (from Wako Pure Chemical Corporation); Lauric Acid (from Tokyo Chemical Industry Co., Ltd.); and Lauric Acid (from Wako Pure Chemical Corporation). A non-limiting example of the commercial products of the salt of medium-chain fatty acid is one available under the trade name of n-Caprylic Acid Sodium Salt (from Tokyo Chemical Industry Co., Ltd.).

The component (B), namely, at least one selected from the group consisting of a medium-chain fatty acid triacylglycerol, a medium-chain fatty acid, and a salt of medium-chain fatty acid may be used alone or in any combination. Assume that these are classified into (i) at least one of medium-chain fatty acid triacylglycerols, (ii) at least one of medium-chain fatty acids, and (iii) at least one of salts of medium-chain fatty acid. In this case, the component (B) preferably includes not less than two of the groups (i), (ii), and (iii), and more preferably includes the groups (i) and (ii) or the groups (i) and (iii). Preferably, the component (B) includes all the groups (i), (ii), and (iii). The component (B), when including any of the preferred combinations of the groups, more advantageously dissolves or solubilizes the component (A) in the solvent and gives a pharmaceutical composition having still more excellent stability.

The pharmaceutical composition according to the first embodiment of the present invention contains the component (B) in a content of preferably 0.1 to 10 weight %, more preferably 0.4 to 8 weight %, and still more preferably 0.5 to 7 weight %, of the total amount (100 weight %) of the pharmaceutical composition. The pharmaceutical composition, when containing the component (B) in a content of not less than 0.1 weight %, allows the component (B) to exhibit a still better function as a solvent for the component (A). The pharmaceutical composition, when containing the component (B) in a content of not more than 10 weight %, has still better stability at low temperatures. The term "content of the component (B)" refers to the total content of all the components (B) in the pharmaceutical composition according to the first embodiment of the present invention.

### Component (C): Nonionic surfactant having an HLB of not less than 10

The component (C) is a nonionic surfactant having an HLB of not less than 10. The component (C) for use in the present invention restrains the separation between the solvent and the component (A), which has been dissolved by the action of the component (B), and allows the preparation to have better emulsion stability and storage stability. Namely, the component (C) functionally adjusts the particle size (particle diameter) of particles upon emulsification and controls coalescence of the particles. The pharmaceutical composition may include each of different components (C) alone or in combination.

As used herein, the term "HLB" or "hydrophile-lipophile balance" is the balance between hydrophilicity and hydrophobicity used generally in the field of surfactants. The HLB employed herein may be any of a theoretical value according to a computational expression, an experimental value, and a numerical value described typically in a catalog.

The nonionic surfactant having an HLB of not less than 10 may be any one usable as preparation additives, and can be appropriately selected according to the intended use. Non-limiting examples of the nonionic surfactant having an HLB of not less than 10 include sucrose fatty acid esters, polyglycerol esters of fatty acids, polyoxyethylene glycerol fatty acid esters, polyoxyethylene sorbitan fatty acid esters (also called polysorbates), polyoxyethylene sorbitol fatty acid esters, polyoxyethylene hydrogenated castor oils, polyoxyethylene sterols, polyoxyethylene alkyl ethers, polyoxyethylene polyoxypropylene alkyl ethers, and polyethylene glycol polyoxyethylene fatty acid esters.

In particular, the component (C) is preferably at least one selected from the group consisting of sucrose fatty acid esters, polyoxyethylene hydrogenated castor oils, and polysorbates, for better emulsion stability and storage stability.

For inhibiting or restraining precipitation, the component (C) is preferably devoid of, or, if any, preferably includes only little amount of sucrose fatty acid esters having an HLB of not less than 10. The pharmaceutical composition according to the first embodiment of the present invention has a content of the sucrose fatty acid esters of typically not more than 0.1 weight % (namely, 0 to 0.1 weight %), preferably not more than 0.01 weight %, and more preferably not more than 0.001 weight %, of the total amount (100 weight %) of the pharmaceutical composition.

Such a sucrose fatty acid ester generally includes not only a monoester, but also other esters such as a diester, a triester, and a polyester and is used as a mixture. The diester, triester, and polyester included in the sucrose fatty acid ester have low solubility in water and tend to precipitate (deposit) at low temperatures. Unfortunately, a high-purity sucrose fatty acid monoester, which less precipitates at low temperatures, is expensive and is impractical to be used as a preparation additive. In this connection, the pharmaceutical composition according to the first embodiment of the present invention is highly stable when existing as an emulsion and storage stability, even when being devoid of sucrose fatty acid esters having an HLB of not less than 10, and contributes to still further reduction of precipitation.

The component (C) may be available as a commercial product. Non-limiting examples of such commercial products of the sucrose fatty acid esters include ones available under the trade names of: DK Ester SS (HLB: about 19), DK Ester F-160 (HLB: 15), DK Ester F-140 (HLB: 13), and DK Ester F-110 (HLB: 11) (each from Dai-ichi Kogyo Seiyaku Co., Ltd.); and SURFHOPE J1616 (HLB: 16) (from Mitsubishi Chemical Foods Corporation). Non-limiting examples of the commercial products of the polyoxyethylene hydrogenated castor oils include ones available under the trade names of: NIKKOL HCO-40 (polyoxyethylene hydrogenated castor oil 40) (HLB: 12.5), NIKKOL HCO-50 (polyoxyethylene hydrogenated castor oil 50) (HLB: 13.5), and NIKKOL HCO-60 (polyoxyethylene hydrogenated castor oil 60) (HLB: 14) (each from Nikko Chemicals Co., Ltd.). A non-limiting example of the commercial products of the polysorbates include one available under the trade name of Polysorbate 80 (HLB: 15) (from Sanyo Chemical Industries, Ltd.).

The pharmaceutical composition according to the first embodiment of the present invention contains the component (C) in a content of preferably 3 to 15 weight %, more preferably 4 to 13 weight %, and still more preferably 5 to 12 weight %, of the total amount (100 weight %) of the pharmaceutical composition. The pharmaceutical composition according to the first embodiment of the present invention, when containing the component (C) in a content of not less than 3 weight %, can restrain the separation of the component (A) and has still better storage stability. The pharmaceutical composition, when containing the component (C) in a content of not more than 15 weight %, is usable without excessive frothing when the pharmaceutical composition is charged into a container or sprayed to the wall of a digestive tract upon gastrointestinal endoscopy. The term "content of the component (C)" refers to the total content of all the components (C) in the pharmaceutical composition according to the first embodiment of the present invention.

### Component (D): a polyhydric alcohol having not less than three hydroxy groups per molecule and/or a poly(oxyalkylene) glycol

The component (D) is a polyhydric alcohol having not less than three hydroxy groups per molecule and/or a poly(oxyalkylene) glycol. The component (D) advantageously lowers the cloud point of the pharmaceutical composition according to the first embodiment of the present invention and reduces affinity between water and the nonionic surfactant having an HLB of not less than 10. The pharmaceutical composition according to the first embodiment of the present invention, when containing the component (C) and the component (D) in a specific weight ratio, can have a cloud point controlled within a specific range. This contributes to better emulsion stability and storage stability under cold conditions. The pharmaceutical composition may include each of different components (D) alone or in combination.

The component (D) may be any one usable as a preparation additive, and is appropriately selectable according to the intended use. Non-limiting examples of the polyhydric alcohol having not less than three hydroxy groups per molecule include sugar alcohols such as glycerol, maltose, D-sorbitol, maltitol, D-mannitol, and xylitol. Among them, glycerol, D-sorbitol, and D-mannitol are preferred, because they have both the actions of lowering the cloud point and appropriately controlling the viscosity of the preparation.

Non-limiting examples of the poly(oxyalkylene) glycol include diethylene glycol, triethylene glycol, polyethylene glycols (also called "macrogols"), and polypropylene glycols. Among them, macrogol 400 is preferred, because it has both the actions of lowering the cloud point and appropriately controlling the viscosity of the preparation.

The component (D) is preferably selected from sugar alcohols, more preferably selected from D-sorbitol and D-mannitol, and is still more preferably D-mannitol, because these sugar alcohols allow the pharmaceutical composition to have a still lower cloud point and to have excellent storage stability at temperatures in a wide range.

The component (D) may be available as commercial products. A non-limiting example of the commercial products of glycerol is one available under the trade name of Concentrated Glycerol (from Kao Corporation). A non-limiting example of the commercial products of D-sorbitol is one available under the trade name of D-Sorbitol (Japanese Pharmacopoeia) (from Junsei Chemical Co., Ltd.). A non-limiting example of the commercial products of D-mannitol is one available under the trade name of D-Mannitol (from Towa Chemical Industry Co., Ltd.). A non-limiting example of the commercial products of macrogol 400 is one available under the trade name of Macrogol 400 (from Sanyo Chemical Industries, Ltd.).

The pharmaceutical composition according to the first embodiment of the present invention contains the component (D) in a content of preferably 3 to 20 weight %, more preferably 5 to 18 weight %, and still more preferably 8 to 15 weight %, of the total amount (100 weight %) of the pharmaceutical composition. The preparation, when containing the component (D) in a content of not less than 3 weight %, can more effectively have a lower cloud point. The preparation, when containing the component (D) in a content of not more than 20 weight %, is appropriately controlled in viscosity by the action of component (D), while it has an effectively lowered cloud point. The term "content of the component (D)" refers to the total content of all the components (D) in the pharmaceutical composition according to the first embodiment of the present invention.

The weight ratio of content of the component (C) to that of the component (D) [component (C) / component (D)] is preferably 0.2 to 5.0, more preferably 0.4 to 4.0, and still more preferably 0.6 to 3.0 in the pharmaceutical composition. The pharmaceutical composition according to the first embodiment of the present invention, when having such a weight ratio within the range, can more easily have a cloud point controlled within the range of 30°C to 75°C.

### Component (E): Water

The component (E) is water. The component (E) may be any water, but is preferably purified water. The pharmaceutical composition according to the first embodiment of the present invention contains the water in a content of preferably not less than 60.0 weight %, more preferably not less than 70.0 weight %, and still more preferably not less than 75.0 weight %, of the total amount (100 weight %) of the pharmaceutical composition. This is preferred for better emulsion stability.

### Other Components

The pharmaceutical composition according to the first embodiment of the present invention may further include one or more components (other components) other than the components (A) to (E), within ranges not adversely affecting advantageous effects of the present invention. Non-limiting examples of the other components include components generally used in pharmaceutical compositions. More specifically, non-limiting examples of the other components include a stabilizer, a pH regulator, an antifoaming agent, a thickener, a preservative, a buffer, and an antiseptic agent.

The stabilizer is preferably at least one selected from the group consisting of benzoic acid, p-hydroxybenzoic acid, and a salt or an alkyl ester thereof. The stabilizer also advantageously functions or serves as a buffer, a pH regulator, and/or an antiseptic agent.

Non-limiting examples of the stabilizer include benzoic acid, p-hydroxybenzoic acid, a salt of benzoic acid, a salt of p-hydroxybenzoic acid, an alkyl ester of benzoic acid, and an alkyl ester of p-hydroxybenzoic acid. The salts of benzoic acid or p-hydroxybenzoic acid may be any pharmacologically acceptable salts, but are preferably sodium salts, potassium salts, and other alkali metal salts. The alkyl esters of benzoic acid or alkyl esters of p-hydroxybenzoic acid may be any pharmacologically acceptable esters between benzoic acid or p-hydroxybenzoic acid and an alcohol, but are preferably pharmacologically acceptable esters between benzoic acid or p-hydroxybenzoic acid and an alcohol having a C₁-C₄ alkyl moiety. The stabilizer usable herein may be any one usable in preparations. A non-limiting example of such other stabilizers is sodium edetate. In particular, the stabilizer for use herein is preferably selected from benzoic acid, sodium benzoate, methyl p-hydroxybenzoate, ethyl p-hydroxybenzoate, propyl p-hydroxybenzoate, and butyl p-hydroxybenzoate. The pharmaceutical composition may include each of different stabilizers alone or in combination.

The pharmaceutical composition according to the first embodiment of the present invention may contain the stabilizer in a content as benzoic acid of preferably 0.01 to 2.0 weight %, more preferably 0.05 to 1.5 weight %, and still more preferably 0.3 to 0.9 weight %, of the total amount (100 weight %) of the pharmaceutical composition. The preparation, when containing the stabilizer in a content of not less than 0.01 weight %, can have still better storage stability. The pharmaceutical composition, when having a content of the stabilizer of not more than 2.0 weight %, can resist the precipitation of the component (A) even at low temperatures and can more satisfactorily maintain its transparent appearance. The term "content of the stabilizer" refers to the total content of all the stabilizers in the pharmaceutical composition according to the first embodiment of the present invention.

The pH regulator may be any pH regulator usable in preparations, but is preferably selected typically from citric acid buffers, phosphoric acid buffers, hydrochloric acid, lactic acid, sodium hydroxide, and sodium hydrogencarbonate. Among them, citric acid buffers are preferred.

The antifoaming agent may be any antifoaming agent usable in preparations, and may be used as a mixture of not less than two antifoaming agents. In particular, the antifoaming agent is preferably selected from silicones, more preferably selected from dimethylpolysiloxane silicones such as dimethylpolysiloxanes and mixtures of a dimethylpolysiloxane and silicon dioxide, and particularly preferably selected from dimethylpolysiloxanes. These are preferred for restraining frothing and for surely providing a good view field.

The pharmaceutical composition according to the first embodiment of the present invention may contain the antifoaming agent in any content, but in a content of preferably 0.0001 to 0.01 weight %, more preferably 0.0005 to 0.007 weight %, and still more preferably 0.0007 to 0.005 weight %, of the total amount (100 weight %) of the pharmaceutical composition. This is preferred for restraining frothing and for surely providing a good view field. The term "content of the antifoaming agent" refers to the total content of all the antifoaming agents in the pharmaceutical composition according to the first embodiment of the present invention.

The thickener may be any thickener usable in preparations, but is preferably selected typically from carrageenan, methylcellulose, carboxymethylcellulose, guar gum, and pectin. The pharmaceutical composition according to the first embodiment of the present invention may contain the thickener in any content, but the content is selected within the range of 0.01 to 5 weight % of the total amount (100 weight %) of the pharmaceutical composition. This configuration is preferred for controlling the falling-down rate of the pharmaceutical composition within a desired range when the pharmaceutical composition is sprayed to the wall of a digestive tract.

Non-limiting examples of the preservative include sorbic acid and benzalkonium chloride. The pharmaceutical composition according to the first embodiment of the present invention may include each of these preservatives alone or in combination in an appropriate amount.

The pharmaceutical composition according to the first embodiment of the present invention is preferably an emulsion. The pharmaceutical composition, when existing as an emulsion in which the component (A) homogeneously disperses, highly advantageously restrains or inhibits the contraction of smooth muscles when sprayed to the wall of a digestive tract.

Assume that the pharmaceutical composition according to the first embodiment of the present invention is an emulsion. This emulsion has an average particle size (average diameter of particles) of preferably less than 100 nm, more preferably not more than 70 nm, and still more preferably not more than 50 nm. The pharmaceutical composition according to the first embodiment of the present invention, when having an average particle size of less than 100 nm, has a transparent or slightly whitish appearance. When the pharmaceutical composition is sprayed to the wall of a digestive tract typically upon gastrointestinal endoscopy, the pharmaceutical composition surely provides a good view field over the observation area without masking. Assume that the emulsion as the pharmaceutical composition is stored at 4°C for 3 months and then has an average particle size of less than 100 nm. This pharmaceutical composition can be evaluated as a preparation that is highly stable when existing as an emulsion and is highly stable during storage at low temperatures.

The average particle size (in nanometer (nm)) can be measured by preparing a sample liquid, placing the sample liquid in a 10-mm cell, and measuring particle diameters (particle sizes) using a light scattering photometer (trade name Zetasizer Nano ZSP, from Malvern Panalytical).

The pharmaceutical composition according to the first embodiment of the present invention, when existing as an emulsion, may have any emulsion form such as an oil-in-water or water-in-oil emulsion form, but is preferably an oil-in-water emulsion. Assume that the pharmaceutical composition as an oil-in-water emulsion is sprayed to the wall of a digestive tract and comes in contact with an aqueous medium such as a gastrointestinal liquid. In this case, the pharmaceutical composition resists clouding in the observation area. Also assume that particles in the emulsion are controlled to have an average particles size of less than 100 nm and to disperse homogeneously. This emulsion is highly transparent and surely provides a good view field in the observation area.

The pharmaceutical composition according to the first embodiment of the present invention has a light transmittance of preferably not less than 50%, and more preferably not less than 70%. The pharmaceutical composition according to the first embodiment of the present invention, when having a light transmittance of not less than 50%, has high transparency and surely provides a good view field in the observation area without masking, typically when sprayed to the wall of a digestive tract upon gastrointestinal endoscopy. The pharmaceutical composition according to the first embodiment of the present invention, when having a light transmittance of not less than 50% after storage at 4°C for 3 months, can be evaluated as a preparation that is highly stable when existing as an emulsion and is highly stable during storage under low-temperature conditions.

The light transmittance (%) can be measured by preparing a sample liquid, placing the sample liquid in a 10-mm cell, and measuring the light transmittance of the sample liquid at a measurement wavelength of 550 nm using a UV/VIS spectrophotometer (trade name JASCO V-630, from JASCO Corporation).

The pharmaceutical composition according to the first embodiment of the present invention has a pH of preferably 3.0 to 8.0, and more preferably 3.0 to 7.0. This is preferred for the preparation to have a cloud point controlled within the desired temperature range and to have excellent storage stability. Such a pharmaceutical composition containing the component (A), the component (B), the component (C), and water, when having a lower pH, has a lower cloud point; and, when having a higher pH, has a higher cloud point.

### Method according to the first embodiment of the present invention for stabilizing a pharmaceutical composition

As described above, the pharmaceutical composition according to the first embodiment of the present invention includes the components (A), (B), (C), (D), and (E) and has a cloud point of 30°C to 75°C. Thus, the pharmaceutical composition is highly stable when existing as an emulsion. Specifically, assume that a pharmaceutical composition includes the component (A), the component (B), the component (C), and the component (E). This pharmaceutical composition can have a stabilized emulsified state by incorporating the component (D), adjusting the content each of these components, and making the cloud point to be 30°C to 75°C. As used herein, the method for stabilizing the emulsified state of the pharmaceutical composition is also referred to a "method according to the first embodiment of the present invention for stabilizing a pharmaceutical composition".

### Method according to the first embodiment of the present invention for evaluating the storage stability of pharmaceutical composition

The pharmaceutical composition according to the first embodiment of the present invention has a cloud point of 30°C to 75°C and thereby has excellent storage stability in a wide temperature range. Specifically, according to the first embodiment, the storage stability of a pharmaceutical composition which contains the component (A), the component (B), the component (C), and water can be evaluated by measuring the cloud point of this pharmaceutical composition and evaluating the storage stability using, as an index, the difference between the cloud point and a storage temperature of the pharmaceutical composition. Herein, the method for evaluating the storage stability of a pharmaceutical composition is also referred to as a "method according to the first embodiment of the present invention for evaluating the storage stability of a pharmaceutical composition".

Specifically, the method according to the first embodiment of the present invention for evaluating the storage stability of a pharmaceutical composition can evaluate the storage stability by determining the difference in temperature between the cloud point and the upper limit and/or lower limit of the storage temperatures, and evaluating the storage stability on the basis of the temperature difference. The difference between the cloud point and the upper limit of the storage temperatures ((cloud point) - (storage temperature upper limit)) is preferably ±0°C to +45°C, more preferably +10°C to +40°C, and still more preferably +15°C to +30°C. A pharmaceutical composition, when having a temperature difference within the temperature range, can be evaluated as having excellent emulsion stability and storage stability at the predetermined storage temperatures. A pharmaceutical composition, when having a temperature difference of less than ±0°C, may have lower emulsion stability and storage stability and may become cloudy at a temperature around the upper limit (e.g., 25°C) of the predetermined storage temperatures. A pharmaceutical composition, when having a large temperature difference of more than +45°C, may suffer from creaming and/or separation at a temperature around a low temperature (e.g., 4°C), because this pharmaceutical composition may have a higher lower limit of storage temperatures at which the pharmaceutical composition exhibits excellent storage stability, and may have lower emulsion stability and storage stability at such a low temperature. As used herein, the term "creaming" refers to a phenomenon in which emulsified particles come up or down by the density difference between the oil phase and the aqueous phase and partially thicken.

The temperature difference between the cloud point and the lower limit of the storage temperatures ((cloud point) - (storage temperature lower limit)) is preferably +30°C to +75°C, more preferably +40°C to +70°C, and still more preferably +50°C to +65°C. A pharmaceutical composition, when having a temperature difference within the temperature range, can be evaluated as having excellent emulsion stability and storage stability at the predetermined storage temperatures. A pharmaceutical composition (preparation), if having a temperature difference of less than +30°C, may become cloudy at a temperature around a high temperature (e.g., 25°C), because this pharmaceutical composition may have a lower upper limit of the storage temperatures at which the pharmaceutical composition exhibits excellent storage stability, and may have lower emulsion stability and storage stability at such a high temperature. A pharmaceutical composition, if having a large temperature difference of more than +75°C, may have lower emulsion stability and storage stability and may suffer from creaming and/or separation at a temperature (e.g., 4°C) around the lower limit of the predetermined storage temperatures.

In particular, the method can preferably evaluate a pharmaceutical composition having a cloud point of 30°C to 75°C (preferably 35°C to 70°C, and more preferably 35°C to 65°C). Assume that the storage temperatures are predetermined to be from 1°C to 30°C. In this case, the pharmaceutical composition, when having a cloud point controlled within the temperature range, is highly stable when existing as an emulsion and is highly stable during storage at the storage temperatures. In particular, the method can preferably evaluate a pharmaceutical composition including the component (A), the component (B), the component (C), and the component (E).

The method according to the first embodiment of the present invention for evaluating the storage stability of a pharmaceutical composition enables designing of a pharmaceutical composition predicted to have excellent storage stability and emulsion stability at the storage temperatures, by adjusting the cloud point on the basis of the predetermined storage temperatures. The method thus enables designing of a pharmaceutical composition that is highly stable with time.

The pharmaceutical composition according to the first embodiment of the present invention may be produced by a method as described below.

The pharmaceutical composition according to the first embodiment of the present invention is preferably produced by a method including Steps I and II as follows:
Step I: the step of mixing the component (B), the component (C), and the component (D) and dispersing and emulsifying them in the component (E) to give an emulsion; and
Step II: the step of adding the component (A) to the emulsion from Step I, and dispersing and emulsifying them with heating to give an emulsion.

### Step I

Step I is the step of mixing the component (B), the component (C), the component (D), and the component (E), and dispersing and emulsifying them using a stirrer or mixer such as a homomixer to give an emulsion. The emulsion may further receive ultrasonication or may be emulsified using a high-pressure emulsifier, to be an emulsion including fine, homogeneous particles. Where necessary, one or more other components, such as a stabilizer, may be added in Step I. The preliminary dispersion and emulsification of the component (B), the component (C), and the component (D) in the component (E) can give a preparation that has still better storage stability.

### Step II

Step II is the step of adding the component (A) to the emulsion from Step I in the weight ratios and dispersing and emulsifying them with heating to give an emulsion. The mixture is thoroughly stirred with heating using a mixer such as a homomixer. The heating temperature has only to be 50°C or higher, and is preferably 60°C to 130°C, and more preferably 70°C to 121°C. Where necessary, one or more other components, such as an antifoaming agent, may be added in Step II. The mixture may further receive ultrasonication or may be stirred using a high-pressure emulsifier, to be an emulsion including fine, homogeneous particles.

### Other Steps

The method may further include one or more other steps than Steps I and II. For example, in such another step, the emulsion after Step II may be cooled and combined with a pH regulator to have a pH of 3 to 8. Also in such another step, the emulsion may further receive heat sterilization under heating conditions for regular fat emulsions (110°C to 121°C).

The preparation obtained by the production method has excellent emulsion stability and storage stability even when containing 1-menthol in a high concentration. The preparation includes fine particles whose average particle size is controlled to be smaller than the specific level, thereby has a high light transmittance (%), and is highly transparent.

The pharmaceutical composition according to the first embodiment of the present invention may also be produced by a method as follows.

Initially, the component (B), the component (C), and a stabilizer (for example, benzoic acid and/or a salt thereof) mixes with, and thoroughly disperses in the component (E) using a mixer such as a homomixer. Next, the component (A) mixes with the homogeneous mixture containing the component (B), the component (C), the stabilizer, and the component (E), followed by thorough stirring with heating using a mixer such as a homomixer. Where necessary, the resulting mixture may further receive ultrasonication or may be stirred using a high-pressure emulsifier, to allow particles in the preparation to be homogeneous and fine as much as possible. Then another component (such as a pH adjuster) is added according to necessity. After cooling, the mixture mixes with the component (D) and the component (E) to give a preparation. The prepared preparation may further receive sterilization in an autoclave at 115°C for 30 minutes, to give a product preparation. As used herein, the term "room temperature" refers to a temperature of 1°C to 30°C.

The pharmaceutical composition according to the first embodiment of the present invention has low toxicity and is administrable safely to gastrointestinally administrable various mammals, and can be safely administered particularly to humans.

The pharmaceutical composition according to the first embodiment of the present invention is applicable typically to smooth muscles of a digestive organ (digestive tract) such as esophagus, stomach, duodenum, bile duct, small intestine, large intestine, colon, or rectum. The pharmaceutical composition may be directly sprayed to the wall of a digestive tract through a sprayer or an endoscopic forceps guide, typically upon laparotomic or endoscopic surgery of a digestive organ, upon endoscopic examination and/or treatment of a digestive organ, upon examination and/or treatment by endoscopic retrograde cholangiopancreatography (ERCP) (such as endoscopic sphincterotomy (EST) performed in succession from ERCP examination, extraction of bile duct stones or pancreatic stones by endoscopic papillary balloon dilatation (EPBD), or treatment for improving flow of bile by endoscopic retrograde biliary drainage (EBD or ERBD) or pancreatic duct drainage), upon examination and/or treatment using a capsule endoscope, or upon any other medical care requiring the inhibition of gastrointestinal smooth muscle contraction (such as an activity for easing the cannula intubation by relaxing the duodenal papilla opening). As used herein, the term "upon examination" means and includes at least one of before examination, during examination, and after examination. Also as used herein, the term "upon treatment" means and includes at least one of before treatment, during treatment, and after treatment. Also as used herein, the term "upon surgery" means and includes at least one of before surgery, during surgery, and after surgery.

The pharmaceutical composition according to the first embodiment of the present invention may be administered in a dose of generally preferably 10 to 200 mL, and more preferably 20 to 100 mL, typically to the stomach or large intestine of an adult human, although the dose may vary depending on the administration object and administration site.

To directly administer a certain amount of the pharmaceutical composition according to the first embodiment of the present invention through the sprayer or the endoscopic forceps guide, it is preferable to charge a unit dose of the pharmaceutical composition into an extrusive vessel such as a pre-fillable syringe. A product according to the present invention can be charged and stored in a container such as a bottle, vial, or ampoule. The storage container is preferably a container made from a polyolefin resin, a cyclic polyolefin resin, or a polyester resin.

The pharmaceutical composition according to the first embodiment of the present invention contains 1-menthol (the component (A)), at least one selected from the group consisting of a medium-chain fatty acid triacylglycerol, a medium-chain fatty acid, and a salt ofmedium-chain fatty acid (the component (B)), a nonionic surfactant having an HLB of not less than 10 (the component (C)), a polyhydric alcohol having not less than three hydroxy groups per molecule and a poly(oxyalkylene) glycol (the component (D)), and/or water (the component (E)). The nonionic surfactant having an HLB of not less than 10 (the component (C)) has high hydrophilicity. In contrast, the a polyhydric alcohol having not less than three hydroxy groups per molecule and/or a poly(oxyalkylene) glycol (the component (D)) has high affinity for such a nonionic surfactant as compared with water, has lipophilicity, and lowers the affinity between the nonionic surfactant and water. This property acts to lower the cloud point of the preparation. The pharmaceutical composition according to the first embodiment of the present invention further includes the component (D) in addition to the components (A), (B), (C), and (E). This configuration controls the balance between hydrophilicity and lipophilicity of the nonionic surfactant and more advantageously contributes to better emulsion stability and storage stability. In addition, the pharmaceutical composition according to the first embodiment of the present invention is controlled to have a cloud point of 30°C to 75°C. This allows the preparation to resist precipitation of components such as 1-menthol and a surfactant, to resist breakage of the emulsified state, and to maintain its emulsion stability and storage stability. This can give a pharmaceutical composition that has excellent emulsion stability and storage stability even when containing the component (A) in a high concentration.

### Second Embodiment

A gastrointestinal smooth muscle contraction inhibitor according to the second embodiment of the present invention is mainly used upon endoscopy and/or treatment of a digestive organ (digestive tract) such as stomach or large intestine (herein also referred to as "upon gastrointestinal endoscopy"). The gastrointestinal smooth muscle contraction inhibitor according to the second embodiment of the present invention includes 1-menthol; a medium-chain fatty acid triacylglycerol; a medium-chain fatty acid and/or a salt thereof; at least one selected from the group consisting of a sucrose fatty acid ester, a polyoxyethylene hydrogenated castor oil, and a polysorbate; and at least one selected from the group consisting of benzoic acid, p-hydroxybenzoic acid, and a salt or an alkyl ester thereof.

The present invention also provides, in another aspect of the second embodiment, a method for inhibiting gastrointestinal smooth muscle contraction. This method includes administering to a mammal a pharmaceutical composition, where the pharmaceutical composition includes 1-menthol; a medium-chain fatty acid triacylglycerol; a medium-chain fatty acid and/or a salt thereof; at least one selected from the group consisting of a sucrose fatty acid ester, a polyoxyethylene hydrogenated castor oil, and a polysorbate; and at least one selected from the group consisting of benzoic acid, p-hydroxybenzoic acid, and a salt or an alkyl ester thereof.

The present invention also provides, in still another aspect of the second embodiment, use of a pharmaceutical composition for inhibiting gastrointestinal smooth muscle contraction, where the pharmaceutical composition includes 1-menthol; a medium-chain fatty acid triacylglycerol; a medium-chain fatty acid and/or a salt thereof; at least one selected from the group consisting of a sucrose fatty acid ester, a polyoxyethylene hydrogenated castor oil, and a polysorbate; and at least one selected from the group consisting of benzoic acid, p-hydroxybenzoic acid, and a salt or an alkyl ester thereof.

Herein, 1-menthol is also referred to as a "component (A)"; the medium-chain fatty acid triacylglycerol is also referred to as a "component (b1)"; the a medium-chain fatty acid and/or a salt thereof is also referred to as a "component (b2)"; the at least one selected from the group consisting of a sucrose fatty acid ester, a polyoxyethylene hydrogenated castor oil, and a polysorbate is also referred to as a "component (F)"; the at least one selected from the group consisting of benzoic acid, p-hydroxybenzoic acid, and a salt or an alkyl ester thereof is also referred to as a "component (G)".

Possible components to exist in the gastrointestinal smooth muscle contraction inhibitor according to the second embodiment of the present invention, namely, the component (A), the component (b1), the component (b2), the component (F), the component (G), and other components than the components (A), (b1), (b2), (F), and (G) may be used alone or in combination in each category.

### Component (A): 1-Menthol

The component (A) is 1-menthol. It is considered that 1-menthol binds to a voltage-dependent L-type calcium channel on the cell membrane of a smooth muscle, and this blocks calcium ions from entering the cell, eliminates or minimizes the membrane potential occurrence, and relaxes the smooth muscle. When the gastrointestinal smooth muscle contraction inhibitor according to the second embodiment of the present invention is sprayed to the wall of a digestive tract, 1-menthol permeates through the mucosa and reaches, directly acts upon, and relaxes the smooth muscle to inhibit or restrain the contraction of the muscle.

1-Menthol is a principal component of peppermint oil and is present in the peppermint oil in a content of not less than 30 weight %. The peppermint oil results from steam distillation of peppermint (*Mentha piperita*) or Japanese mint (*Mentha avensis*)*.* Such a 1-menthol-containing material usable herein may be peppermint oil, or mint oil, or another similar oil, but is preferably one that has been highly purified from peppermint oil or another oil typically by fractional distillation. The material for use herein is still more preferably one having a purity of not less than 90 weight %. Such 1-menthol-containing materials are recently also available by synthesis. The 1-menthol for use in the present invention is preferably one that conforms to the specifications for 1-menthol as prescribed in Japanese Pharmacopoeia.

A non-limiting example of commercial products of the component (A) is 1-Menthol (trade name, from The Suzuki Menthol Co., Ltd.).

The gastrointestinal smooth muscle contraction inhibitor according to the second embodiment of the present invention contains the component (A) in a content of 0.01 to 5 weight %, preferably 0.1 to 4 weight %, more preferably from more than 0.8 weight % but not more than 3 weight %, and most preferably 1 to 2 weight %, of the total amount (100 weight %) of the gastrointestinal smooth muscle contraction inhibitor. The gastrointestinal smooth muscle contraction inhibitor, as containing the component (A) in a content of not less than 0.01 weight %, can sufficiently relax smooth muscles of a digestive organ. The gastrointestinal smooth muscle contraction inhibitor, as containing the component (A) in a content of not more than 5 weight %, can serve as a preparation that is stable over a long time without precipitation, even at low temperatures.

### Component (b1): Medium-chain fatty acid triacylglycerol

The component (b1) is a medium-chain fatty acid triacylglycerol. The component (b1) advantageously dissolves or solubilizes the component (A) in a solvent. The component (b1), as coexisting with the component (b2) and the component (G), further stabilizes the preparation and protects the preparation from precipitation at low temperatures, even when the preparation contains the component (A) in a high concentration. The component (b1) is not limited, but is preferably a triacylglycerol derived from each of different C₆-C₁₂ fatty acids alone or in combination. Non-limiting examples of the C₆-C₁₂ fatty acids include caproic acid (C6), caprylic acid (C8), capric acid (C10), and lauric acid (C12). Among them, tricaprylin (glycerol tricaprylate), which is derived from three moieties of a C8 fatty acid alone, is particularly preferred. The gastrointestinal smooth muscle contraction inhibitor may include each of different components (b1) alone or in combination.

The component (b1) is available as commercial products. Non-limiting examples of such commercial products include ones available under the trade names of: COCONARD RK, COCONARD MT, and COCONARD ML (each from Kao Corporation); and PANACET 800 (from NOF Corporation).

The content of component (b1) is 0.1 to 10 times, and preferably 0.2 to 5 times, as much by weight as that of the component (A). The content of component (b1), as being not less than 0.1 times by weight, exhibits the function as a solvent for the component (A). The content of component (b1), as being not more than 10 times by weight, further stabilizes the preparation at low temperatures, because of lowering the proportion of the component (b2). The gastrointestinal smooth muscle contraction inhibitor according to the second embodiment of the present invention contains the component (b1) in a content of typically 0.3 to 1 weight %, preferably 0.4 to 0.9 weight %, and more preferably 0.5 to 0.7 weight %, of the total amount (100 weight %) of the inhibitor. As used herein, the term "content of the component (b1)" refers to the total content of all the components (b1) in the gastrointestinal smooth muscle contraction inhibitor according to the second embodiment of the present invention.

### Component (b2): a medium-chain fatty acid and/or a salt thereof

The component (b2) is a medium-chain fatty acid and/or a salt thereof. The component (b2), together with the component (b1), advantageously dissolves or solubilizes the component (A) in the solvent. The component (b2), as coexisting with the component (b1) and the component (G), further stabilizes the preparation and protects the preparation from precipitation at low temperatures, even when the preparation contains the component (A) in a high concentration. The component (b2) is not limited, but is preferably any of C₆-C₁₂ fatty acids and/or salts thereof. Non-limiting examples of the C₆-C₁₂ fatty acids include caproic acid (C6), caprylic acid (C8), capric acid (C10), and lauric acid (C12). The salt of the medium-chain fatty acid may be any pharmacologically acceptable salt, but is preferably any of alkali metal salts such as sodium salts and potassium salts. Among them, caprylic acid, which contains 8 carbon atoms, and/or an alkali metal salt of caprylic acid (such as sodium caprylate or potassium caprylate) is preferably employed. The gastrointestinal smooth muscle contraction inhibitor may contain each of different components (b2) alone or in combination.

The component (b2) is available as a commercial product. Non-limiting examples of such commercial products include ones available under the trade names of: Hexanoic Acid (from Wako Pure Chemical Corporation); Caprylic acid (from Merck Millipore); Decanoic Acid (from Wako Pure Chemical Corporation); Lauric Acid (from Tokyo Chemical Industry Co., Ltd.); Lauric Acid (from Wako Pure Chemical Corporation); and n-Caprylic Acid Sodium Salt (from Tokyo Chemical Industry Co., Ltd.).

The content of component (b2) is 0.1 to 10 times, and preferably 0.2 to 5 times, as much by weight as that of the component (A). The content of component (b2), as being not less than 0.1 times by weight, exhibits the function as a solvent for the component (A). The content of component (b2), as being not more than 10 times by weight, further stabilizes the preparation at low temperatures. The gastrointestinal smooth muscle contraction inhibitor according to the second embodiment of the present invention contains the component (b2) in a content of typically 0.2 to 1 weight %, preferably 0.4 to 0.9 weight %, and more preferably 0.5 to 0.7 weight %, of the total amount (100 weight %) of the inhibitor. The term "content of the component (b2)" refers to the total content of all the components (b2) in the gastrointestinal smooth muscle contraction inhibitor according to the second embodiment of the present invention.

Component (F): At least one selected from the group consisting of a sucrose fatty acid ester, a polyoxyethylene hydrogenated castor oil, and a polysorbate

The component (F) is at least one selected from the group consisting of a sucrose fatty acid ester, a polyoxyethylene hydrogenated castor oil, and a polysorbate. The component (F) serves as a nonionic surfactant. The component (F) for use in the present invention advantageously inhibits or restrains the separation between the solvent and the component (A), which has been dissolved by the action of the component (b1) and the component (b2), and allows the preparation to have better storage stability. Specifically, the component (F) has the function of regulating diameters of particles upon emulsification and controlling coalescence of the particles. The gastrointestinal smooth muscle contraction inhibitor may contain each of different components (F) alone or in combination.

In particular, the gastrointestinal smooth muscle contraction inhibitor according to the second embodiment of the present invention preferably contains a sucrose fatty acid ester as the component (F). This is preferred for the inhibitor to have still better stability during storage at low temperatures.

In general, such a sucrose fatty acid ester includes not only a monoester, but also other esters such as a diester, a triester, and a polyester and is often used as a mixture. The sucrose fatty acid ester for use in the present invention may contain a sucrose fatty acid monoester in a proportion of preferably not less than 55 weight %, more preferably not less than 60 weight %, and still more preferably not less than 65 weight %, of the total amount of the sucrose fatty acid ester. The sucrose fatty acid ester, when containing the corresponding monoester in a proportion of not less than 55 weight %, has a higher hydrophile-lipophile balance (HLB), thereby can disperse the component (A) more satisfactorily, and allows the preparation to have still better storage stability. Such a sucrose fatty acid ester generally tends to undergo precipitation at low temperatures, because the diester, triester, and polyester have low solubility in water. However, the preparation, as having a ratio of the component (b2) to the component (G) controlled within the specific range, can have still better storage stability. This is probably because the configuration allows the esters to be more readily adsorbed by the oil interface.

The content of the monoester in the sucrose fatty acid ester may be determined typically by the analysis of the sucrose fatty acid ester through high-performance liquid chromatography (HPLC). Specifically, the content of the monoester can be determined by analyzing the sample sucrose fatty acid ester through HPLC, plotting a chromatogram of the test sample using a data processor, measuring peak areas of peaks in the chromatogram corresponding to components in the test sample using an integrator, and determining the monoester content as an area percentage on the basis of the measured peak areas.

The component (F) preferably includes all the sucrose fatty acid ester, the polyoxyethylene hydrogenated castor oil, and the polysorbate, particularly for still better stability during storage.

The component (F) may be available as a commercial product. Non-limiting examples of such commercial products of the sucrose fatty acid esters include ones available under the trade names of: DK Ester SS (monoester content: about 100 weight %) and DK Ester F-10 (monoester content: about 0 weight %) (each from Dai-ichi Kogyo Seiyaku Co., Ltd.); and SURFHOPE J1616 (monoester content: about 70 weight %, from Mitsubishi Chemical Foods Corporation). A non-limiting example of the commercial products of the polyoxyethylene hydrogenated castor oils is one available under the trade name of NIKKOL HCO-60 (polyoxyethylene hydrogenated castor oil 60; from Nikko Chemicals Co., Ltd.). A non-limiting example of the commercial products of the polysorbates is one available under the trade name of Polysorbate 80 (from Sanyo Chemical Industries, Ltd.).

The content of component (F) is 0.1 to 10 times, and preferably 0.5 to 5.0 times, as much by weight as the total amount of the component (A), the component (b1), and the component (b2). The content of component (F), as being not less than 0.1 times by weight, allows the gastrointestinal smooth muscle contraction inhibitor according to the second embodiment of the present invention to have excellent storage stability, because this component restrains or inhibits the separation of the component (A) in the inhibitor. The content of component (F), as being content of not more than 10 times by weight, allows the inhibitor to be usable without excessive frothing, when the inhibitor is charged into a container or sprayed to the wall of a digestive tract upon gastrointestinal endoscopy. The gastrointestinal smooth muscle contraction inhibitor according to the second embodiment of the present invention may contain the component (F) in any content, but typically preferably in a content of 0.1 to 20 weight %, and more preferably 0.5 to 15 weight %, of the total amount (100 weight %) of the inhibitor. The term "content of the component (F)" refers to the total content of all the components (F) in the gastrointestinal smooth muscle contraction inhibitor according to the second embodiment of the present invention.

The component (F) in the gastrointestinal smooth muscle contraction inhibitor according to the second embodiment of the present invention may contain a sucrose fatty acid ester in a content of preferably 15 to 40 weight %, and more preferably 20 to 30 weight %; may contain a polyoxyethylene hydrogenated castor oil in a content of preferably 40 to 80 weight %, and more preferably 50 to 70 weight %; and may contain a polysorbate in a content of preferably 3 to 20 weight %, and more preferably 5 to 15 weight %. The content of the sucrose fatty acid ester, the polyoxyethylene hydrogenated castor oil, or the polysorbate refers to the content relative to a total amount of all the components (F) in the gastrointestinal smooth muscle contraction inhibitor according to the second embodiment of the present invention. The component (F) preferably contains these ingredients in proportions within the ranges, for providing a more stable emulsified state.

Component (G): At least one selected from the group consisting of benzoic acid, p-hydroxybenzoic acid, and a salt or an alkyl ester thereof

The component (G) is at least one selected from the group consisting of benzoic acid, p-hydroxybenzoic acid, a salt or an alkyl ester thereof. The component (G) serves as a stabilizer, and, in the present invention, used in combination with the component (b2) in a specific blending ratio. Thus, the component (G) restrains or inhibits the separation of the component (A) and allows the preparation to have better storage stability. The component (G) functions not only as a stabilizer, but also as a buffer, pH regulator, and/or antiseptic agent.

The category component (G) includes benzoic acid, p-hydroxybenzoic acid, a salt of benzoic acid, a salt of p-hydroxybenzoic acid, an alkyl ester of benzoic acid, and an alkyl ester of p-hydroxybenzoic acid. The salts of benzoic acid or p-hydroxybenzoic acid may be any pharmacologically acceptable salts, but are preferably alkali metal salts such as sodium salts or potassium salts. The alkyl esters of benzoic acid or p-hydroxybenzoic acid may be any pharmacologically acceptable esters between benzoic acid or p-hydroxybenzoic acid and an alcohol, but are preferably pharmacologically acceptable esters between benzoic acid or p-hydroxybenzoic acid and an alcohol having a C₁-C₄ alkyl moiety. The stabilizers usable herein may be any ones usable in preparations. In particular, the component (G) for use herein is preferably selected from benzoic acid, sodium benzoate, methyl p-hydroxybenzoate, ethyl p-hydroxybenzoate, propyl p-hydroxybenzoate, and butyl p-hydroxybenzoate. The gastrointestinal smooth muscle contraction inhibitor may include each of different components (G) alone or in combination.

The gastrointestinal smooth muscle contraction inhibitor according to the second embodiment of the present invention contains the component (G) in a content as benzoic acid of 0.01 to 2.0 weight %, more preferably 0.05 to 1.5 weight %, and still more preferably 0.3 to 0.9 weight %, of the total amount (100 weight %) of the inhibitor. The inhibitor (preparation), as containing the component (G) in a content of not less than 0.01 weight %, can have still better stability. The inhibitor, as containing the component (G) in a content of not more than 2.0 weight %, allows the component (A) to resist precipitation and maintains its transparent appearance. The term "content of the component (G)" refers to the total content of all the components (G) in the gastrointestinal smooth muscle contraction inhibitor according to the second embodiment of the present invention.

The weight ratio of the component (b2) to the component (G) [component (b2) / component (G)] is 1/5 to 10/1, preferably 1/5 to 5/1, more preferably 1/3 to 3/1, and still more preferably 1/2 to 2/1. The inhibitor as a preparation, as containing these components in a weight ratio within the range, has higher storage stability and resists precipitation at low temperatures. This is probably because as follows. The component (G), as coexisting with the component (b2) in a specific compositional ratio, provides emulsification, exerts a synergistic effect with the component (F), and maintains a stable emulsified state even when the component (A) is present in a high concentration.

### Other Components

The gastrointestinal smooth muscle contraction inhibitor according to the second embodiment of the present invention may further include one or more "other components" than the components (A), (b1), (b2), (F), and (G), within ranges not adversely affecting advantageous effects of the present invention. Non-limiting examples of the other components include water, a pH regulator, an antifoaming agent, a thickener, a stabilizer other than the component (G), and a preservative.

The water may be any water, but is preferably purified water. The gastrointestinal smooth muscle contraction inhibitor according to the second embodiment of the present invention may contain the water in a content of preferably not less than 60.0 weight %, more preferably not less than 70.0 weight %, and still more preferably not less than 80.0 weight %, of the total amount (100 weight %) of the inhibitor. This is preferred for better emulsion stability.

The pH regulator may be any pH regulator usable in preparations, but is preferably selected typically from citric acid buffers, phosphoric acid buffers, hydrochloric acid, lactic acid, sodium hydroxide, and sodium hydrogencarbonate. Among them, citric acid buffers are preferred.

The antifoaming agent may be any antifoaming agent usable in preparations, and may be used as a mixture of not less than two antifoaming agents. In particular, the antifoaming agent is preferably selected from silicones, more preferably selected from dimethylpolysiloxane silicones such as dimethylpolysiloxanes and mixtures of a dimethylpolysiloxane and silicon dioxide, and particularly preferably selected from dimethylpolysiloxanes. These are preferred for restraining frothing and for surely providing a good view field.

The gastrointestinal smooth muscle contraction inhibitor according to the second embodiment of the present invention may contain the antifoaming agent in any content, but may contain in a content of preferably 0.0001 to 0.01 weight %, more preferably 0.0005 to 0.007 weight %, and still more preferably 0.0007 to 0.005 weight %, of the total amount of (100 weight %) of the inhibitor. This is preferred for restraining frothing and for surely providing a good view field. The term "content of the antifoaming agent" refers to the total content of all the antifoaming agents in the gastrointestinal smooth muscle contraction inhibitor according to the second embodiment of the present invention.

The thickener may be any thickener usable in preparations, but is preferably selected typically from carrageenan, methylcellulose, carboxymethylcellulose, guar gum, and pectin. The gastrointestinal smooth muscle contraction inhibitor according to the second embodiment of the present invention may contain the thickener in any content, but may contain in a content selected within the range of 0.01 to 5 weight % of the total amount (100 weight %) of the inhibitor, for regulating the falling-down rate to a desired rate when the inhibitor is sprayed to the wall of a digestive tract.

A non-limiting example of the stabilizer (namely, stabilizer other than the components (G)) is sodium edetate. Non-limiting examples of the preservative include sorbic acid and benzalkonium chloride. Each of these components may be added in an appropriate amount to the gastrointestinal smooth muscle contraction inhibitor according to the second embodiment of the present invention.

The gastrointestinal smooth muscle contraction inhibitor according to the second embodiment of the present invention is preferably an emulsion. Assume that the inhibitor exists as an emulsion in which the component (A) homogeneously disperses. This inhibitor, when sprayed to the wall of a digestive tract, advantageously restrains or inhibits the contraction of smooth muscles of the digestive organ.

Assume that the gastrointestinal smooth muscle contraction inhibitor according to the second embodiment of the present invention is an emulsion. In this emulsion, particles have an average particle size of preferably less than 100 nm, more preferably not more than 70 nm, and still more preferably not more than 50 nm. The gastrointestinal smooth muscle contraction inhibitor according to the second embodiment of the present invention, when having an average particle size of less than 100 nm, has a transparent or slightly whitish appearance. The inhibitor, when sprayed to the wall of a digestive tract typically upon gastrointestinal endoscopy, surely provides a good view field of the observation area without masking.

The average particle size (in nanometer (nm)) can be measured by preparing a sample liquid, placing the sample liquid in a 10-mm cell, and measuring particle diameters using a light scattering photometer (trade name Zetasizer Nano ZSP, from Malvern Panalytical).

The gastrointestinal smooth muscle contraction inhibitor according to the second embodiment of the present invention may be in any emulsion form such as an oil-in-water or water-in-oil emulsion form, but is preferably an oil-in-water emulsion. The inhibitor, when being an oil-in-water emulsion, resists clouding in the observation area, when the inhibitor is sprayed to the wall of a digestive tract and comes in contact with an aqueous medium such as a gastrointestinal liquid. In this emulsion, particles are controlled to have an average particle size of less than 100 nm and to disperse homogeneously. The emulsion is therefore highly transparent and surely provides a good view field in the observation area.

The gastrointestinal smooth muscle contraction inhibitor according to the second embodiment of the present invention has a light transmittance of preferably not less than 50%, and more preferably not less than 70%. The gastrointestinal smooth muscle contraction inhibitor according to the second embodiment of the present invention, when having a light transmittance of not less than 50%, has high transparency, and surely provides a good view field in the observation area without masking, typically when sprayed to the wall of a digestive tract upon gastrointestinal endoscopy.

The light transmittance (%) can be measured by preparing a sample liquid, placing the sample liquid in a 10-mm cell, and measuring the light transmittance at a measurement wavelength of 550 nm using a UV/VIS spectrophotometer (trade name JASCO V-630, from JASCO Corporation).

The gastrointestinal smooth muscle contraction inhibitor according to the second embodiment of the present invention may have any pH, but has a pH of preferably 3.0 to 8.0, and more preferably 4.0 to 7.0. This is preferred for avoiding irritation to the digestive organ.

The gastrointestinal smooth muscle contraction inhibitor according to the second embodiment of the present invention may be produced by a production method as described below.

The gastrointestinal smooth muscle contraction inhibitor according to the second embodiment of the present invention is preferably produced by a method including Steps III and IV as follows:
Step (III): the step of mixing and dispersing the component (b1), the component (b2), the component (F), and the component (G) in purified water and emulsifying them to give an emulsion; and
Step (IV): the step of adding component (A) to the emulsion from Step III and dispersing and emulsifying them with heating to give an emulsion.

### Step III

This step is the step of mixing the component (b1), the component (b2), the component (F), and the component (G) with purified water, and dispersing and emulsifying them using a mixer such as a homomixer to give an emulsion. The emulsion may further receive ultrasonication or may be emulsified using a high-pressure emulsifier, to be an emulsion including fine, homogeneous particles. Where necessary, one or more other components, such as a stabilizer, may be added in Step III. The preliminary dispersion and emulsification of the component (b1), the component (b2), the component (F), and the component (G) in purified water can give a preparation that has still better storage stability.

### Step IV

This step is the step of adding the component (A) to the emulsion from Step III in the weight ratios, and dispersing and emulsifying them with heating to give an emulsion. The mixture is thoroughly stirred with heating using a mixer such as a homomixer. The heating temperature has only to be 50°C or higher, and is preferably 60°C to 130°C, and more preferably 70°C to 121°C. The mixture may further receive ultrasonication or may be stirred using a high-pressure emulsifier, to be an emulsion including fine, homogeneous particles.

### Other Steps

After Step IV, the emulsion may be cooled and then combined with a pH regulator to have a pH of 3 to 8. This gives an emulsion as a gastrointestinal smooth muscle contraction inhibitor according to the second embodiment of the present invention. However, the pH adjustment with a pH adjuster may be performed before the cooling. The emulsion may further receive heat sterilization under heating conditions for regular fat emulsions (110°C to 121°C).

The preparation obtained by the production method has excellent stability at low temperatures even when containing l-menthol in a high concentration. This preparation includes fine particles whose average particle size is controlled to be smaller than the specific level, has a high light transmittance (%), and is highly transparent.

Alternatively, the gastrointestinal smooth muscle contraction inhibitor according to the second embodiment of the present invention may be produced by the following method.

Initially, the component (A) is dissolved in the component (b1), the component (b2), and the component (G). The dissolution may be performed at room temperature or with heating. Next, the component (F) is added to, and dispersed thoroughly in purified water using a mixer such as a homomixer. The resulting mixture is combined with the above-prepared homogeneous mixture including the component (A), the component (b1), the component (b2), and the component (G) and stirred thoroughly using a mixer such as a homomixer. Where necessary, the mixture may further receive ultrasonication or may be dispersed typically using a high-pressure emulsifier, to allow particles in the preparation to be homogeneous and fine as much as possible. The prepared preparation then receives sterilization in an autoclave at 115°C, 30 minutes. As used herein, the term "room temperature" refers to a temperature of from 1°C to 30°C.

The gastrointestinal smooth muscle contraction inhibitor according to the second embodiment of the present invention has low toxicity and is administrable safely to gastrointestinally administrable various mammals, and can be safely administered particularly to humans.

The gastrointestinal smooth muscle contraction inhibitor according to the second embodiment of the present invention is applicable typically to smooth muscles of a digestive organ (digestive tract) such as esophagus, stomach, duodenum, bile duct, small intestine, large intestine, colon, or rectum. The gastrointestinal smooth muscle contraction inhibitor may be directly sprayed to the wall of a digestive tract through a sprayer or an endoscopic forceps guide, typically upon laparotomic or endoscopic surgery of a digestive organ, upon endoscopic examination and/or treatment of a digestive organ, upon examination and/or treatment by endoscopic retrograde cholangiopancreatography (ERCP) (such as endoscopic sphincterotomy (EST) performed in succession from ERCP examination, extraction of bile duct stones or pancreatic stones by endoscopic papillary balloon dilatation (EPBD), or treatment for improving flow of bile by endoscopic retrograde biliary drainage (EBD or ERBD) or pancreatic duct drainage), upon examination and/or treatment using a capsule endoscope, or upon any other medical care requiring the inhibition of gastrointestinal smooth muscle contraction (such as an activity for relaxing the duodenal papilla opening to ease the cannula intubation). As used herein, the term "upon examination" means and includes at least one of before examination, during examination, and after examination. Also as used herein, the term "upon treatment" means and includes at least one of before treatment, during treatment, and after treatment. Also as used herein, the term "upon surgery" means and includes at least one of before surgery, during surgery, and after surgery.

The gastrointestinal smooth muscle contraction inhibitor according to the second embodiment of the present invention may be administered in a dose of generally preferably 10 to 200 mL, and more preferably 20 to 100 mL, typically to the stomach or large intestine of an adult human, although the dose may vary depending on the administration object and administration site.

To directly administer a certain amount of the gastrointestinal smooth muscle contraction inhibitor according to the second embodiment of the present invention through the sprayer or the endoscopic forceps guide, it is preferable to charge a unit dose of the inhibitor into an extrusive vessel such as a pre-fillable syringe. A product according to the present invention can be charged and stored in a container such as a bottle, vial, or ampoule. The storage container is preferably a container made from a polyolefin resin, a cyclic polyolefin resin, or a polyester resin.

The gastrointestinal smooth muscle contraction inhibitor according to the second embodiment of the present invention contains l-menthol (the component (A)), a medium-chain fatty acid triacylglycerol (the component (b1)), a medium-chain fatty acid and/or a salt thereof (the component (b2)), at least one selected from the group consisting of sucrose fatty acid esters, polyoxyethylene hydrogenated castor oils, and polysorbates (the component (F)), and at least one selected from the group consisting of benzoic acid, p-hydroxybenzoic acid, and a salt or an alkyl ester thereof (the component (G)). In the inhibitor, the weight ratio of the component (b2) to the component (G) is controlled within the specific range. Thus, the inhibitor resists precipitation of components such as l-menthol and a surfactant and can maintain its stability as a preparation even at low temperatures. This allows the gastrointestinal smooth muscle contraction inhibitor to have excellent low-temperature stability even when containing the component (A) in a high concentration.

### Examples

The present invention will be illustrated in further detail with reference to several examples below. It should be noted, however, that the examples are by no means intended to limit the scope of the present invention. All the proportions or amounts in the following tables are percentages by weight, unless otherwise specified. The symbol "-" in the tables refers to "not incorporated" for a component; and refers to "unmeasured" for a measurement data.

### First Embodiment

### Example 1-1

To 1500 mL of water, were added 10.0 g of polysorbate 80 (trade name Polysorbate 80, Japanese Pharmacopoeia, from Sanyo Chemical Industries, Ltd.) and 0.2 g of dimethylpolysiloxane (trade name Q7-9120, Japanese Pharmaceutical Excipients, from DOW CORNING),, followed by emulsification using a homomixer (at a liquid temperature of 80°C) to give an emulsion. The emulsion further mixed with water up to a total volume of 2000 mL and yielded a dimethylpolysiloxane solution. Separately, to 1000 mL of water, were added 200 g of D-sorbitol (trade name D-Sorbitol, Japanese Pharmacopoeia, from Junsei Chemical Co., Ltd.), 12.0 g of benzoic acid (trade name Benzoic Acid, Japanese Pharmacopoeia, from FUSHIMI Pharmaceutical Co., Ltd.), 24.0 g of tricaprylin (trade name COCONARD RK, Japanese Pharmaceutical Codex, from Kao Corporation), 4.0 g of caprylic acid (trade name Caprylic acid, Ph Eur, from Merck Millipore), 14.0 g of polysorbate 80 (trade name Polysorbate 80, Japanese Pharmacopoeia, from Sanyo Chemical Industries, Ltd.), and 160.0 g of polyoxyethylene hydrogenated castor oil 60 (trade name NIKKOL HCO-60, Japanese Pharmaceutical Excipients, from Nikko Chemicals Co., Ltd.). These were dispersed using a homomixer to give a dispersion. The dispersion mixed with 32.6 g of l-menthol (trade name 1-Menthol, Japanese Pharmacopoeia, from The Suzuki Menthol Co., Ltd.), followed by emulsification at a liquid temperature of 80°C for 10 minutes using a homomixer to give an emulsion. The emulsion mixed with 200 mL of the dimethylpolysiloxane solution, followed by emulsification using a homomixer. After cooling, the resulting emulsion mixed with water up to a total volume of 2000 mL and yielded a pharmaceutical composition.

### Example 1-2

A pharmaceutical composition was prepared according to a formulation similar to that in Example 1-1, except for using the benzoic acid (trade name Benzoic Acid, Japanese Pharmacopoeia, from FUSHIMI Pharmaceutical Co., Ltd.) in an amount of 6.0 g, further incorporating 6.0 g of sodium benzoate (trade name Sodium Benzoate, Japanese Pharmacopoeia, from FUSHIMI Pharmaceutical Co., Ltd.), and adding citric acid hydrate (trade name Citric Acid Hydrate, from KOMATSUYA CORPORATION) to adjust pH.

### Example 1-3

A pharmaceutical composition was prepared according to a formulation similar to that in Example 1-2, except for using the polyoxyethylene hydrogenated castor oil 60 (trade name NIKKOL HCO-60, Japanese Pharmaceutical Excipients, from Nikko Chemicals Co., Ltd.) in an amount of 140.0 g.

### Example 1-4

Materials were added to 1000 mL of water and dispersed therein using a homomixer. The materials were 6.0 g of sodium benzoate (trade name Sodium Benzoate, Japanese Pharmacopoeia, from FUSHIMI Pharmaceutical Co., Ltd.), 6.0 g of benzoic acid (trade name Benzoic Acid, Japanese Pharmacopoeia, from FUSHIMI Pharmaceutical Co., Ltd.), 24.0 g of tricaprylin (trade name COCONARD RK, Japanese Pharmaceutical Codex, from Kao Corporation), 4.0 g of caprylic acid (trade name Caprylic acid, Ph Eur, from Merck Millipore), 15.0 g of polysorbate 80 (trade name Polysorbate 80, Japanese Pharmacopoeia, from Sanyo Chemical Industries, Ltd.), and 160.0 g of polyoxyethylene hydrogenated castor oil 60 (trade name NIKKOL HCO-60, Japanese Pharmaceutical Excipients, from Nikko Chemicals Co., Ltd.). The resulting liquid mixed with 32.6 g of l-menthol (trade name l-Menthol, Japanese Pharmacopoeia, from The Suzuki Menthol Co., Ltd.), followed by emulsification at a liquid temperature of 80°C for 10 minutes using a homomixer to give an emulsion. After cooling, the emulsion mixed with citric acid hydrate (trade name Citric Acid Hydrate, from KOMATSUYA CORPORATION) to adjust pH, and further mixed with water up to a total volume of 1800 mL. To 225 mL of this liquid were added 12.5 g of D-mannitol (trade name D-Mannitol, Japanese Pharmacopoeia, from Towa Kagaku Co., Ltd.) and dissolved. The liquid further mixed with water up to a total volume of 250 mL, and yielded a pharmaceutical composition.

### Example 1-5

A pharmaceutical composition was prepared according to a formulation similar to that in Example 1-4, except for using the D-mannitol (trade name D-Mannitol, Japanese Pharmacopoeia, from Towa Kagaku Co., Ltd.) in an amount of 25.0 g.

### Comparative Example 1-1

A pharmaceutical composition was prepared according to a formulation similar to that in Example 1-4, except for not using the D-mannitol (trade name D-Mannitol, Japanese Pharmacopoeia, from Towa Kagaku Co., Ltd.).

### Comparative Example 1-2

A pharmaceutical composition was prepared according to a formulation similar to that in Example 1-4, except for using the D-mannitol (trade name D-Mannitol, Japanese Pharmacopoeia, from Towa Kagaku Co., Ltd.) in an amount of 2.5 g.

### Comparative Example 1-3

A pharmaceutical composition was prepared according to a formulation similar to that in Example 1-4, except for not using the D-mannitol (trade name D-Mannitol, Japanese Pharmacopoeia, from Towa Kagaku Co., Ltd.), and further incorporating 24 mL of propylene glycol (trade name propylene glycol, specific gravity (20°C): 1.0375, JIS Special Grade, from Wako Pure Chemical Corporation).

### Evaluations

Each 20 mL of the pharmaceutical compositions obtained in Example 1-1 to 1-5 and Comparative Example 1-1 to 1-3 were placed in a 20-mL transparent glass vial, the vial was hermetically sealed, and stored at 4°C or 25°C for 3 months. The pharmaceutical compositions underwent following five experiments, and the results are presented in Table 1.

### Experiment 1-1: Cloud Point

The cloud points of the pharmaceutical compositions obtained in the examples and the comparative examples at the beginning of the experiment were measured. Each 20 mL of the pharmaceutical compositions obtained in the examples and the comparative examples, a stirring bar, and a temperature sensor (trade name SK-1250MCII, from Sato Keiryoki MFG. Co., Ltd.) were placed in a 20-mL transparent glass vial (diameter: 30 mm) with a sticky label as a mark at the bottom, and the vial was hermetically sealed. The contents in the vial were gradually heated on a water bath (trade name STW-181, from SANYO) with stirring using a stirrer (trade name ATLAS-30G, from Koike Precision Instruments) (stirring conditions: 4 at speed range High). Each sample liquid was visually observed, and the temperature at the time point when the mark at the vial bottom could not be seen by cloud or turbid was measured as a cloud point.

### Experiment 1-2: Light Transmittance (%)

Each of the pharmaceutical compositions obtained in the examples and the comparative examples was placed in a 10-mm cell and used as a sample liquid. The sample liquid was evaluated for light transmittance by measurement at a wavelength of 550 nm using a UV/VIS spectrophotometer (trade name JASCO V-630, from JASCO Corporation).

### Experiment 1-3: pH

The pH was measured according to the pH measurement method described in General Tests of Japanese Pharmacopoeia. Each about 5 mL of the emulsion compositions obtained in the examples and the comparative examples were placed in a test tube, and each sample with stirring was evaluated for pH by measurement (measurement time: 3 minutes) using a pH meter (trade name F-21, from HORIBA, Ltd.), which had been calibrated with a neutral phosphate pH standard solution and a phthalate pH standard solution.

### Experiment 1-4: Average Particle Diameter (nm)

Each of the pharmaceutical compositions obtained in the examples and the comparative examples was placed in a 10-mm cell and used as a sample liquid. The sample liquid was evaluated for average particle size by measurement using a light scattering photometer (trade name Zetasizer Nano ZSP, from Malvern Panalytical).

### Experiment 1-5: Appearance

Each of the pharmaceutical compositions obtained in the examples and the comparative examples was placed in a 20-mL transparent glass vial and visually observed through the vial. Each sample was evaluated for appearance according to the evaluation criteria as follows:

### Appearance Evaluation Criteria

Good: transparent liquid;
Fair: slightly white or whitish transparent liquid, but is practically usable;
Poor: cloudy liquid with creaming and/or separation, or including precipitates.

### [Table 1]

**TABLE 1**

| | Component | Examples | | | | | | | | | | Comparative Examples | | | | | |
|---|---|---|---|---|---|---|---|---|---|---|---|---|---|---|---|---|---|
| | | 1-1 | | 1-2 | | 1-3 | | 1-4 | | 1-5 | | 1-1 | | 1-2 | | 1-3 | |
| (A) | I-Menthol | 1.6 | | 1.6 | | 1.6 | | 1.6 | | 1.6 | | 1.6 | | 1.6 | | 1.6 | |
| (B) | Tricaprylin | 1.2 | | 1.2 | | 1.2 | | 1.2 | | 1.2 | | 1.2 | | 1.2 | | 1.2 | |
| | Caprylic acid | 0.2 | | 0.2 | | 0.2 | | 0.2 | | 0.2 | | 0.2 | | 0.2 | | 0.2 | |
| (C) | Polyoxyethylene hydrogenated castor oil 60 | 8.0 | | 8.0 | | 7.0 | | 8.0 | | 8.0 | | 8.0 | | 8.0 | | 8.0 | |
| | Polysorbate 80 | 0.75 | | 0.75 | | 0.75 | | 0.75 | | 0.75 | | 0.75 | | 0.75 | | 0.75 | |
| (D) | D-Sorbitol | 10.0 | | 10.0 | | 10.0 | | - | | - | | - | | - | | - | |
| | D-Mannitol | - | | - | | - | | 5.0 | | 10.0 | | - | | 1.0 | | - | |
| (E) | Purified water | adeq. | | adeq. | | adeq. | | adeq. | | adeq. | | adeq. | | adeq. | | adeq. | |
| Others | Propylene glycol | - | | - | | - | | - | | - | | - | | - | | 10.0 | |
| | Dimethylpolysiloxane | 0.001 | | 0.001 | | 0.001 | | - | | - | | - | | - | | - | |
| | Sodium benzoate | - | | 0.3 | | 0.3 | | 0.3 | | 0.3 | | 0.3 | | 0.3 | | 0.3 | |
| | Benzoic acid | 0.6 | | 0.3 | | 0.3 | | 0.3 | | 0.3 | | 0.3 | | 0.3 | | 0.3 | |
| | Citric acid hydrate | - | | adeq. | | adeq. | | adeq. | | adeq. | | adeq. | | adeq. | | adeq. | |
| Total | | 100.00 | | 100.00 | | 100.00 | | 100.00 | | 100.00 | | 100.00 | | 100.00 | | 100.00 | |
| Cloud point | | 68 | | 70 | | 70 | | 67 | | 50 | | 80 | | 77 | | 85 | |
| Temperature difference between cloud point and storage temperature (upper limit) | | 38 | | 40 | | 40 | | 37 | | 20 | | 50 | | 47 | | 55 | |
| Ratio of component (C) to component (D) | | 0.875 | | 0.875 | | 0.775 | | 1.750 | | 0.875 | | - | | 8.750 | | 0.875 | |
| Measurement at storage at 25°C for 3 months | | Initial | 3 mos. | Initial | 3 mos. | Initial | 3 mos. | Initial | 3 mos. | Initial | 3 mos. | Initial | 3 mos. | Initial | 3 mos. | Initial | 3 mos. |
| | Light transmittance (%) | 97.3 | 96.5 | 97.5 | 97.5 | 96.6 | 96.5 | 97.1 | 97.1 | 97.2 | 97.1 | 96.6 | 82.0 | 96.7 | 95.5 | 97.5 | 80.7 |
| | pH | 3.14 | 3.03 | 4.03 | 4.01 | 4.00 | 3.98 | 5.05 | 5.01 | 5.16 | 5.13 | 4.99 | 4.82 | 4.96 | 4.88 | 4.99 | 4.90 |
| | Average particle size (nm) | 28 | - | 29 | 29 | 30 | - | 21 | 22 | 24 | 25 | 22 | 25 | 21 | 22 | 31 | 36 |
| | Appearance | Good | Good | Good | Good | Good | Good | Good | Good | Good | Good | Good | Good | Good | Good | Good | Good |
| Measurement at storage at 4°C for 3 months | | Initial | 3 mos. | Initial | 3 mos. | Initial | 3 mos. | Initial | 3 mos. | Initial | 3 mos. | Initial | 3 mos. | Initial | 3 mos. | Initial | 3 mos. |
| | Light transmittance (%) | 97.3 | 76.6 | 97.5 | 94.3 | 96.6 | 89.3 | 97.1 | 55.0 | 97.2 | 97.0 | 96.6 | 0.2 | 96.7 | 0.2 | 97.5 | 0.4 |
| | pH | 3.14 | 3.12 | 4.03 | 4.02 | 4.00 | 4.00 | 5.05 | 5.05 | 5.16 | 5.17 | 4.99 | 4.93 | 4.96 | 4.95 | 4.99 | 4.97 |
| | Average particle size (nm) | 28 | - | 29 | 28 | 30 | - | 21 | 67 | 24 | 25 | 22 | 1455 | 21 | 1305 | 31 | 7352 |
| | Appearance | Good | Fair | Good | Good | Good | Good | Good | Fair | Good | Good | Good | Poor | Good | Poor | Good | Poor |

| | | | | | | | | | | | | | | | | | |
|---|---|---|---|---|---|---|---|---|---|---|---|---|---|---|---|---|---|
| The symbol "-" in "Average particle size (nm)" in the table refers to that this parameter was unmeasured. | | | | | | | | | | | | | | | | | |

The results in Table 1 demonstrate that the pharmaceutical compositions obtained in Examples 1-2, 1-3, and 1-5 according to the first embodiment had transparent appearances (evaluation: Good) and did not suffer from significant variations in light transmittance (%), pH, and average particle size (nm) after storage at 4°C and 25°C for 3 months (3 mos.). The pharmaceutical composition obtained in Example 1-1 according to the first embodiment, which had a pH of about 3, had a slightly whitish appearance (evaluation: Fair), but had a practically acceptable light transmittance of 76.6%, after storage at 4°C for 3 months. The pharmaceutical composition obtained in Example 1-4 according to the first embodiment, which included D-mannitol in a proportion of 5 weight %, had a slightly whitish appearance (evaluation: Fair), but had a practically acceptable light transmittance of 55.0%. The pharmaceutical compositions according to the examples had a cloud point of 50°C to 70°C, and had a temperature difference between the cloud point and the storage temperature (upper limit) of +20°C to +40°C. In contrast, Comparative Example 1-1 is based on the formulation devoid of the component (D), Comparative Example 1-2 is based on the formulation using the component (D) in a content of 1 weight %, and Comparative Example 1-3 is based on the formulation using, instead of the component (D), propylene glycol, which has two hydroxy groups per molecule. The resulting pharmaceutical compositions according to Comparative Examples 1-1, 1-2, and 1-3 suffered from creaming, had a significantly lowered light transmittance (%), lost optical transparency, and had an increased average particle size due to aggregation and/or coalescence of emulsified particles, after storage at 4°C for 3 months. The pharmaceutical compositions according to the comparative examples had a cloud point of 77°C to 85°C, higher than 75°C, and had a temperature difference between the cloud point and the storage temperature (upper limit) of +47°C to +55°C. These data demonstrate that the pharmaceutical compositions according to the first embodiment, as controlled to have a cloud point of 30°C to 75°C, have better emulsion stability and storage stability particularly in a low-temperature environment. The data also demonstrate that the pharmaceutical compositions, as controlled to have a weight ratio of the component (C) to the component (D) within a specific range, can be controlled to have a cloud point of 30°C to 75°C.

### Examples 1-6 to 1-9, Comparative Example 1-4

Pharmaceutical compositions were prepared according to the formulations given in Table 2, stored at 4°C for 1 month, and visually observed through a 20-mL transparent glass in which each pharmaceutical composition was contained. The appearances of each sample were evaluated according to the following evaluation criteria. The evaluation results are presented in Table 2 and FIG. 1.

### Appearance Evaluation Criteria

Good: transparent liquid;
Fair: slightly white or whitish transparent liquid, but is practically usable;
Poor: cloudy liquid with creaming and/or separation, or including precipitates.

### [Table 2]

**TABLE 2**

| | Component | Examples | | | | Comp. Ex. |
|---|---|---|---|---|---|---|
| | | 1-6 | 1-7 | 1-8 | 1-9 | 1-4 |
| (A) | I-Menthol | 1.6 | 1.6 | 1.6 | 1.6 | 1.6 |
| (B) | Tricaprylin | 1.2 | 1.2 | 1.2 | 1.2 | 1.2 |
| | Caprylic acid | 0.2 | 0.2 | 0.2 | 0.2 | 0.2 |
| (C) | Polyoxyethylene hydrogenated castor oil 60 | 8.0 | 8.0 | 7.0 | 8.0 | 8.0 |
| | Polysorbate 80 | 0.75 | 0.75 | 0.75 | 0.75 | 0.75 |
| (D) | D-Sorbitol | 10.0 | - | - | - | - |
| | D-Mannitol | - | 10.0 | - | - | - |
| | Macrogol 400 | - | - | 10.0 | - | - |
| | Cone. glycerol | - | - | - | 10.0 | - |
| (E) | Purified water | adeq. | adeq. | adeq. | adeq. | adeq. |
| Others | Dimethylpolysiloxane | 0.001 | 0.001 | 0.001 | 0.001 | 0.001 |
| | Sodium benzoate | 0.3 | 0.3 | 0.3 | 0.3 | 0.3 |
| | Benzoic acid | 0.3 | 0.3 | 0.3 | 0.3 | 0.3 |
| Total | | 100.00 | 100.00 | 100.00 | 100.00 | 100.00 |
| Appearance | | Good | Good | Fair | Fair | Poor |

The results are presented in Table 2 and FIG. 1. The pharmaceutical composition of Comparative Example 1-4, according to the formulation devoid of the component (D), had a cloudy appearance without transparency. In contrast, the pharmaceutical composition of Example 1-6 including D-sorbitol, and the pharmaceutical composition of Example 1-7 including D-mannitol had transparent appearances. The pharmaceutical composition of Example 1-8 including macrogol 400 and the pharmaceutical composition of Example 1-9 including concentrated glycerol had a slightly whitish transparent appearance, but had a sufficient transparency at a practically usable level. As described above, the comparison between the examples including the component (D) and Comparative Example 1-4 (according to the formulation devoid of the component (D)) demonstrate that the presence of the component (D) allows the pharmaceutical compositions to have better emulsion stability and storage stability.

The components described in Table 2 are as follows:
Macrogol 400: trade name Macrogol 400, Japanese Pharmacopoeia, from Sanyo Chemical Industries, Ltd.
Conc. glycerol: trade name Concentrated Glycerol, Japanese Pharmacopoeia, from Kao Corporation

### Experiment 1-6: Long-term Storage Stability

The pharmaceutical compositions obtained in the examples and the comparative examples were evaluated for long-term storage stability. The pharmaceutical compositions after storage for 6 months, 9 months, and 12 months from the beginning of the experiments underwent five experiments as follows. The results are presented in Table 3 and Table 4. For Examples 1-2, 1-3, and 1-4, the long-term storage stability was evaluated only on storage at 25°C. As used herein, the term "long term" refers to a period of time of 6 months from the beginning of the experiment.

### [Table 3]

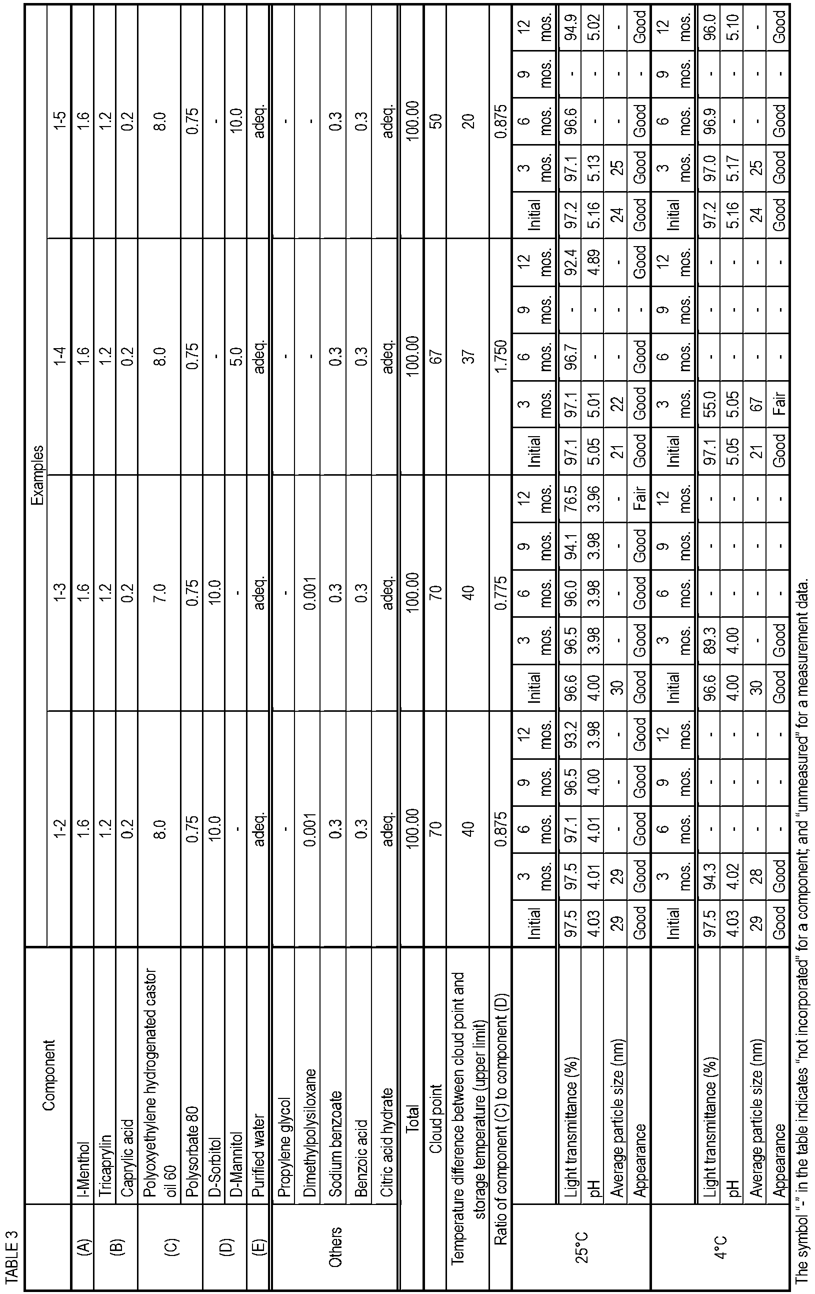

### [Table 4]

**TABLE 4**

| | Component | Comparative Examples | | | | | | | | | | | | | | |
|---|---|---|---|---|---|---|---|---|---|---|---|---|---|---|---|---|
| | | 1-1 | | | | | 1-2 | | | | | 1-3 | | | | |
| (A) | I-Menthol | 1.6 | | | | | 1.6 | | | | | 1.6 | | | | |
| (B) | Tricaprylin | 1.2 | | | | | 1.2 | | | | | 1.2 | | | | |
| | Caprylic acid | 0.2 | | | | | 0.2 | | | | | 0.2 | | | | |
| (C) | Polyoxyethylene hydrogenated castor oil 60 | 8.0 | | | | | 8.0 | | | | | 8.0 | | | | |
| | Polysorbate 80 | 0.75 | | | | | 0.75 | | | | | 0.75 | | | | |
| (D) | D-Sorbitol | - | | | | | - | | | | | - | | | | |
| | D-Mannitol | - | | | | | 1.0 | | | | | - | | | | |
| (E) | Purified water | adeq. | | | | | adeq. | | | | | adeq. | | | | |
| Others | Propylene glycol | - | | | | | - | | | | | 10.0 | | | | |
| | Dimethylpolysiloxane | - | | | | | - | | | | | - | | | | |
| | Sodium benzoate | 0.3 | | | | | 0.3 | | | | | 0.3 | | | | |
| | Benzoic acid | 0.3 | | | | | 0.3 | | | | | 0.3 | | | | |
| | Citric acid hydrate | adeq. | | | | | adeq. | | | | | adeq. | | | | |
| Total | | 100.00 | | | | | 100.00 | | | | | 100.00 | | | | |
| Cloud point | | 80 | | | | | 77 | | | | | 85 | | | | |
| Temperature difference between cloud point and storage temperature (upper limit) | | 50 | | | | | 47 | | | | | 55 | | | | |
| Ratio of component (C) to component (D) | | - | | | | | 8.750 | | | | | 0.875 | | | | |
| 25°C | | Initial | 3 mos. | 6 mos. | 9 mos. | 12 mos. | Initial | 3 mos. | 6 mos. | 9 mos. | 12 mos. | Initial | 3 mos. | 6 mos. | 9 mos. | 12 mos. |
| | Light transmittance (%) | 96.6 | 82.0 | 22.4 | - | 1.24 | 96.7 | 95.5 | 88.7 | - | 3.57 | 97.5 | 80.7 | 49.2 | - | 42.7 |
| | pH | 4.99 | 4.82 | - | - | 4.77 | 4.96 | 4.88 | - | - | 4.79 | 4.99 | 4.90 | - | - | 4.82 |
| | Average particle size (nm) | 22 | 25 | - | - | - | 21 | 22 | - | - | - | 31 | 36 | - | - | - |
| | Appearance | Good | Good | Poor | - | Poor | Good | Good | Good | - | Poor | Good | Good | Fair | - | Fair |
| 4°C | | Initial | 3 mos. | 6 mos. | 9 mos. | 12 mos. | Initial | 3 mos. | 6 mos. | 9 mos. | 12 mos. | Initial | 3 mos. | 6 mos. | 9 mos. | 12 mos. |
| | Light transmittance (%) | 96.6 | 0.20 | 0.20 | - | 0.20 | 96.7 | 0.20 | 0.20 | - | 0.21 | 97.5 | 0.40 | 0.26 | - | 0.26 |
| | pH | 4.99 | 4.93 | - | - | 4.85 | 4.96 | 4.95 | - | - | 4.89 | 4.99 | 4.97 | - | - | 4.91 |
| | Average particle size (nm) | 22 | 1455 | - | - | - | 21 | 1305 | - | - | - | 31 | 7352 | - | - | - |
| | Appearance | Good | Poor | Poor | - | Poor | Good | Poor | Poor | - | Poor | Good | Poor | Poor | - | Poor |

| | | | | | | | | | | | | | | | | |
|---|---|---|---|---|---|---|---|---|---|---|---|---|---|---|---|---|
| The symbol "-" in the table indicates "not incorporated" for a component; and "unmeasured" for a measurement data. | | | | | | | | | | | | | | | | |

The data in Table 3 demonstrate that the pharmaceutical compositions according to Examples 1-2, 1-3, 1-4, and 1-5 were stable even after storage at 25°C for 12 months. These results indicate that the pharmaceutical compositions have excellent long-term storage stability. The pharmaceutical composition of Example 1-5 presented a transparent appearance and was stable with little variations in both the light transmittance (%) and pH, as compared with the data at the beginning of the experiments, even after storage at 4°C for 12 months. These results indicate that the pharmaceutical composition of Example 1-5 has excellent long-term storage stability also at low temperatures.

In contrast, the pharmaceutical compositions according to Comparative Examples 1-1 and 1-2 as indicated in Table 4 presented a transparent appearance after storage at 25°C for 3 months, but became cloudy and had a significantly lower light transmittance (%) after storage at 25°C for 12 months. Thus, these pharmaceutical compositions were found to have poor stability. After storage at 4°C for 12 months, all the pharmaceutical compositions according to the comparative examples had a lower light transmittance (%) down to about 0.2%. These indicate that the pharmaceutical compositions according to Comparative Examples 1-1, 1-2, and 1-3 have poor long-term storage stability.

### Second Embodiment

### Example 2-1

To 1500 mL of water, were added 10.0 g of polysorbate 80 (trade name Polysorbate 80, from Sanyo Chemical Industries, Ltd.) and 0.2 g of dimethylpolysiloxane (trade name Q7-9120, from DOW CORNING), followed by emulsification using a homomixer (at a liquid temperature of 80°C). The resulting liquid mixed with water up to a total volume of 2000 mL and yielded a dimethylpolysiloxane solution. Separately, materials were added to 1000 mL of water and dispersed therein using a homomixer, where the materials were 30.0 g of a sucrose fatty acid ester (trade name SURFHOPE J1616, monoester content: about 70 weight %, from Mitsubishi Chemical Foods Corporation), 10.0 g of sodium caprylate (trade name n-Caprylic Acid Sodium Salt, from Tokyo Chemical Industry Co., Ltd.), 8.0 g of benzoic acid (trade name Benzoic Acid, from FUSHIMI Pharmaceutical Co., Ltd.), 4.0 g of caprylic acid (trade name Caprylic acid, from Merck Millipore), 10.0 g of tricaprylin (trade name COCONARD RK, from Kao Corporation), 14.0 g of polysorbate 80 (trade name Polysorbate 80, from Sanyo Chemical Industries, Ltd.), and 60.0 g of polyoxyethylene hydrogenated castor oil 60 (trade name NIKKOL HCO-60, from Nikko Chemicals Co., Ltd.). The resulting liquid mixed with 32.0 g of l-menthol (trade name l-Menthol, from The Suzuki Menthol Co., Ltd.), followed by emulsification at a liquid temperature of 80°C for 10 minutes using a homomixer. This further mixed with 200 mL of the dimethylpolysiloxane solution, followed by emulsification using a homomixer. After cooling, the liquid mixed with citric acid hydrate to have a pH of 5, mixed with water up to a total volume of 2000 mL, and yielded an emulsion composition.

### Example 2-2

An emulsion composition was prepared according to a formulation similar to that in Example 2-1, except for using the benzoic acid (trade name Benzoic Acid, from FUSHIMI Pharmaceutical Co., Ltd.) in an amount of 10.0 g.

### Example 2-3

Materials were added to 1000 mL of water and dispersed therein using a homomixer, where the materials were: 22.5 g of a sucrose fatty acid ester (trade name DK Ester SS, monoester content: about 100 weight %, from Dai-ichi Kogyo Seiyaku Co., Ltd.), 7.5 g of a sucrose fatty acid ester (trade name DK Ester F-10, monoester content: about 0 weight %, from Dai-ichi Kogyo Seiyaku Co., Ltd.), 10.0 g of sodium caprylate (trade name n-Caprylic Acid Sodium Salt, from Tokyo Chemical Industry Co., Ltd.), 8.0 g of benzoic acid (trade name Benzoic Acid, from FUSHIMI Pharmaceutical Co., Ltd.), 4.0 g of caprylic acid (trade name Caprylic acid, from Merck Millipore), 10.0 g of tricaprylin (trade name COCONARD RK, from Kao Corporation), 15.0 g of polysorbate 80 (trade name Polysorbate 80, from Sanyo Chemical Industries, Ltd.), and 60.0 g of polyoxyethylene hydrogenated castor oil 60 (trade name NIKKOL HCO-60, from Nikko Chemicals Co., Ltd.). The resulting liquid mixed with 32.6 g of l-menthol (trade name l-Menthol, from The Suzuki Menthol Co., Ltd.), followed by emulsification at a liquid temperature of 80°C for 10 minutes using a homomixer. After cooling, the liquid mixed with citric acid hydrate to have a pH of 5, further mixed with water up to a total volume of 2000 mL, and yielded an emulsion composition.

### Example 2-4

An emulsion composition was prepared according to a formulation similar to that in Example 2-3, except for using the sucrose fatty acid ester (trade name DK Ester SS, monoester content: about 100 weight %, from Dai-ichi Kogyo Seiyaku Co., Ltd.) in an amount of 24.0 g and the sucrose fatty acid ester (trade name DK Ester F-10, monoester content: about 0 weight %, from Dai-ichi Kogyo Seiyaku Co., Ltd.) in an amount of 6.0 g.

### Example 2-5

An emulsion composition was prepared according to a formulation similar to that in Example 2-3, except for using the sucrose fatty acid ester (trade name DK Ester SS, monoester content: about 100 weight %, from Dai-ichi Kogyo Seiyaku Co., Ltd.) in an amount of 25.5 g and the sucrose fatty acid ester (trade name DK Ester F-10, monoester content: about 0 weight %, from Dai-ichi Kogyo Seiyaku Co., Ltd.) in an amount of 4.5 g.

### Example 2-6

An emulsion composition was prepared according to a formulation similar to that in Example 2-1, except for using, instead of the sucrose fatty acid ester, another sucrose fatty acid ester (trade name DK Ester SS, monoester content: about 100 weight %, from Dai-ichi Kogyo Seiyaku Co., Ltd.), and using the l-menthol (trade name l-Menthol, from The Suzuki Menthol Co., Ltd.) in an amount of 32.6 g.

### Example 2-7

An emulsion composition was prepared according to a formulation similar to that in Example 2-6, except for using the tricaprylin (trade name COCONARD RK, from Kao Corporation) in an amount of 12.0 g and the sodium caprylate (trade name n-Caprylic Acid Sodium Salt, from Tokyo Chemical Industry Co., Ltd.) in an amount of 6.0 g, and using no citric acid hydrate.

### Example 2-8

An emulsion composition was prepared according to a formulation similar to that in Example 2-6, except for using the tricaprylin (trade name COCONARD RK, from Kao Corporation) in an amount of 14.0 g and the sodium caprylate (trade name n-Caprylic Acid Sodium Salt, from Tokyo Chemical Industry Co., Ltd.) in an amount of 6.0 g, and using no citric acid hydrate.

### Example 2-9

An emulsion composition was prepared according to a formulation similar to that in Example 2-6, except for using the sucrose fatty acid ester (trade name DK Ester SS, monoester content: about 100 weight %, from Dai-ichi Kogyo Seiyaku Co., Ltd.) in an amount of 24.0 g and the benzoic acid (trade name Benzoic Acid, from FUSHIMI Pharmaceutical Co., Ltd.) in an amount of 10.0 g.

### Example 2-10

An emulsion composition was prepared according to a formulation similar to that in Example 2-9, except for using the sucrose fatty acid ester (trade name DK Ester SS, monoester content: about 100 weight %, from Dai-ichi Kogyo Seiyaku Co., Ltd.) in an amount of 26.0 g.

### Example 2-11

An emulsion composition was prepared according to a formulation similar to that in Example 2-9, except for using the sucrose fatty acid ester (trade name DK Ester SS, monoester content: about 100 weight %, from Dai-ichi Kogyo Seiyaku Co., Ltd.) in an amount of 30.0 g.

### Example 2-12

An emulsion composition was prepared according to a formulation similar to that in Example 2-7, except for using the benzoic acid (trade name Benzoic Acid, from FUSHIMI Pharmaceutical Co., Ltd.) in an amount of 10.0 g.

### Example 2-13

An emulsion composition was prepared according to a formulation similar to that in Example 2-8, except for using the benzoic acid (trade name Benzoic Acid, from FUSHIMI Pharmaceutical Co., Ltd.) in an amount of 10.0 g.

### Example 2-14

An emulsion composition was prepared according to a formulation similar to that in Example 2-11, except for using the benzoic acid (trade name Benzoic Acid, from FUSHIMI Pharmaceutical Co., Ltd.) in an amount of 12.0 g, and using no citric acid hydrate.

### Example 2-15

An emulsion composition was prepared according to a formulation similar to that in Example 2-14, except for using the benzoic acid (trade name Benzoic Acid, from FUSHIMI Pharmaceutical Co., Ltd.) in an amount of 6.0 g, further incorporating 6.0 g of sodium benzoate (trade name Sodium Benzoate, from FUSHIMI Pharmaceutical Co., Ltd.), and adding citric acid hydrate to have a pH of 5.

### Example 2-16

An emulsion composition was prepared according to a formulation similar to that in Example 2-15, except for using no citric acid hydrate.

### Example 2-17

An emulsion composition was prepared according to a formulation similar to that in Example 2-6, except for using 10.0 g of lauric acid (trade name Lauric Acid, from Wako Pure Chemical Corporation), using none of sodium caprylate, caprylic acid, and benzoic acid, and further incorporating 8.0 g of sodium benzoate (trade name Sodium Benzoate, from FUSHIMI Pharmaceutical Co., Ltd.).

### Comparative Example 2-1

To 1500 mL of water, were added 10.0 g of polysorbate 80 (trade name Polysorbate 80, from Sanyo Chemical Industries, Ltd.) and 0.2 g of dimethylpolysiloxane (trade name Q7-9120, from DOW CORNING), followed by emulsification using a homomixer (at a liquid temperature of 80°C). The resulting liquid mixed with water up to a total volume of 2000 mL, and yielded a dimethylpolysiloxane solution. Separately, materials were added to 1000 mL of water and dispersed therein using a homomixer, where the materials were: 30.0 g of a sucrose fatty acid ester (trade name SURFHOPE J1616, monoester content: about 70 weight %, from Mitsubishi Chemical Foods Corporation), 6.0 g of sodium caprylate (trade name n-Caprylic Acid Sodium Salt, from Tokyo Chemical Industry Co., Ltd.), 40.0 g of sodium benzoate (trade name Sodium Benzoate, from FUSHIMI Pharmaceutical Co., Ltd.), 14.0 g of tricaprylin (trade name COCONARD RK, from Kao Corporation), 14.0 g of polysorbate 80 (trade name Polysorbate 80, from Sanyo Chemical Industries, Ltd.), and 60.0 g of polyoxyethylene hydrogenated castor oil 60 (trade name NIKKOL HCO-60, from Nikko Chemicals Co., Ltd.). The resulting liquid mixed with 32.0 g of 1-menthol (trade name 1-Menthol, from The Suzuki Menthol Co., Ltd.), followed by emulsification at a liquid temperature of 80°C for 10 minutes using a homomixer. The liquid further mixed with 200 mL of the dimethylpolysiloxane solution, followed by emulsification using a homomixer. After cooling, the liquid mixed with citric acid hydrate to have a pH of 4.8, further mixed with water up to a total volume of 2000 mL, and yielded an emulsion composition.

### Comparative Example 2-2

An emulsion composition was prepared according to a formulation similar to that in Comparative Example 2-1, except for using the sodium caprylate (trade name n-Caprylic Acid Sodium Salt, from Tokyo Chemical Industry Co., Ltd.) in an amount of 10.0 g and the tricaprylin (trade name COCONARD RK, from Kao Corporation) in an amount of 10.0 g, using no sodium benzoate (trade name Sodium Benzoate, from FUSHIMI Pharmaceutical Co., Ltd.), and adding citric acid hydrate to have a pH of 5.

### Evaluations

Each 20 mL of the emulsion compositions obtained in Examples 2-1 to 2-17 and Comparative Examples 2-1 and 2-2 were placed in a 20-mL transparent glass vial, the vial was hermetically sealed, and stored at 4°C or 25°C for 3 months. The emulsion compositions at the beginning of experiments (initial values) and after storage for 3 months were evaluated by the following five experiments. The results are given in Tables 5 and 6.

### Experiment 2-1: 1-Menthol Residual Rate (%)

The emulsion compositions obtained in the examples and the comparative examples were evaluated for 1-menthol residual rate. The 1-menthol residual rate was measured by gas chromatography (using a gas chromatograph, trade name GC-2010, from Shimadzu Corporation) in conformity to the menthol assay for mint oil prescribed in Japanese Pharmacopoeia.

### Experiment 2-2: Light Transmittance (%)

Each of the emulsion compositions obtained in the examples and the comparative examples was placed in a 10-mm cell and used as a sample liquid. The sample liquid was evaluated for light transmittance (%) by measurement at a wavelength of 550 nm using a UV/VIS spectrophotometer (trade name JASCO V-630, from JASCO Corporation).

### Experiment 2-3: pH

The pH was measured according to the pH measurement method described in General Tests of Japanese Pharmacopoeia. Each about 5 mL of the emulsion compositions obtained in the examples and the comparative examples were placed in a test tube, and the pH of each sample with stirring was measured (measurement time: 3 minutes) using a pH meter (trade name F-21, from HORIBA, Ltd.), which had been calibrated with a neutral phosphate pH standard solution and a phthalate pH standard solution.

### Experiment 2-4: Average Particle Diameter (nm)

Each of the emulsion compositions obtained in the examples and the comparative examples was placed in a 10-mm cell and used as a sample liquid. The sample liquid was evaluated for average particle size (nm) by measurement using a light scattering photometer (trade name Zetasizer Nano ZSP, from Malvern Panalytical).

### Experiment 2-5: Appearance

Each of the emulsion compositions obtained in the examples and the comparative examples was placed in a 20-mL transparent glass vial, visually observed through the vial, and evaluated for appearance.

### [Table 5]

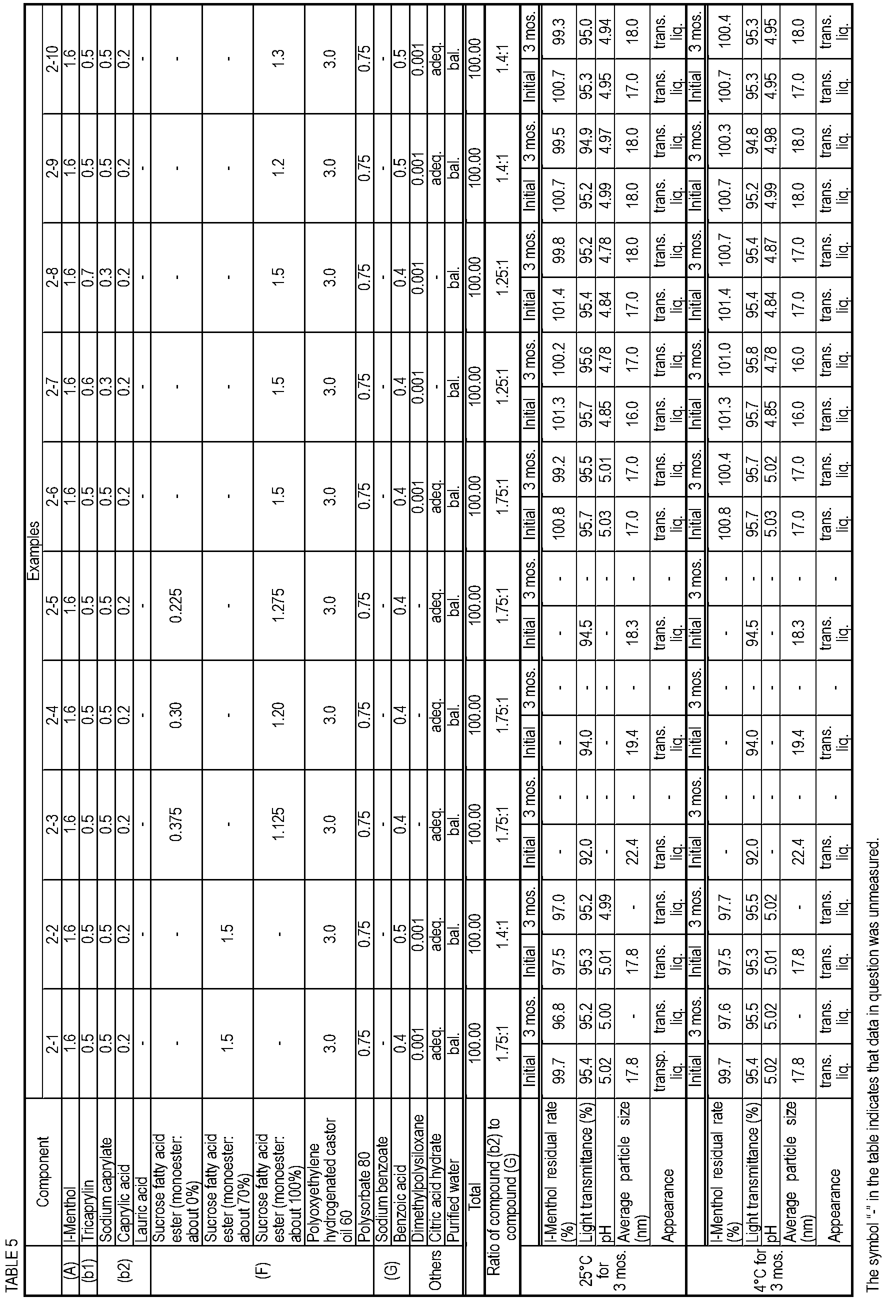

### [Table 6]

**TABLE 6**

| | Component | Examples | | | | | | | | | | | | | | Comparative Examples | | | |
|---|---|---|---|---|---|---|---|---|---|---|---|---|---|---|---|---|---|---|---|
| | | 2-11 | | 2-12 | | 2-13 | | 2-14 | | 2-15 | | 2-16 | | 2-17 | | 2-1 | | 2-2 | |
| (A) | I-Menthol | 1.6 | | 1.6 | | 1.6 | | 1.6 | | 1.6 | | 1.6 | | 1.6 | | 1.6 | | 1.6 | |
| (b1) | Tricaprylin | 0.5 | | 0.6 | | 0.7 | | 0.5 | | 0.5 | | 0.5 | | 0.5 | | 0.7 | | 0.5 | |
| (b2) | Sodium caprylate | 0.5 | | 0.3 | | 0.3 | | 0.5 | | 0.5 | | 0.5 | | - | | 0.3 | | 0.5 | |
| | Caprylic acid | 0.2 | | 0.2 | | 0.2 | | 0.2 | | 0.2 | | 0.2 | | - | | - | | - | |
| | Lauric acid | - | | - | | - | | - | | - | | - | | 0.5 | | - | | - | |
| (F) | Sucrose fatty acid ester (monoester: about 0%) | - | | - | | - | | - | | - | | - | | - | | - | | - | |
| | Sucrose fatty acid ester (monoester: about 70%) | | | | | - | | - | | - | | - | | - | | 1.5 | | 1.5 | |
| | Sucrose fatty acid ester (monoester: about 100%) | 1.5 | | 1.5 | | 1.5 | | 1.5 | | 1.5 | | 1.5 | | 1.5 | | - | | - | |
| | Polyoxyethylene hydrogenated castor oil 60 | 3.0 | | 3.0 | | 3.0 | | 3.0 | | 3.0 | | 3.0 | | 3.0 | | 3.0 | | 3.0 | |
| | Polysorbate 80 | 0.75 | | 0.75 | | 0.75 | | 0.75 | | 0.75 | | 0.75 | | 0.75 | | 0.75 | | 0.75 | |
| (G) | Sodium benzoate | - | | - | | - | | - | | 0.3 | | 0.3 | | 0.4 | | 2.0 | | - | |
| | Benzoic acid | 0.5 | | 0.5 | | 0.5 | | 0.6 | | 0.3 | | 0.3 | | - | | - | | - | |
| Others | Dimethylpolysiloxane | 0.001 | | 0.001 | | 0.001 | | 0.001 | | 0.001 | | 0.001 | | 0.001 | | 0.001 | | 0.001 | |
| | Citric acid hydrate | adeq. | | - | | - | | - | | adeq. | | - | | adeq. | | adeq. | | adeq. | |
| | Purified water | bal. | | bal. | | bal. | | bal. | | bal. | | bal. | | bal. | | bal. | | bal. | |
| Total | | 100.00 | | 100.00 | | 100.00 | | 100.00 | | 100.00 | | 100.00 | | 100.00 | | 100.00 | | 100.00 | |
| Ratio of compound (b2) to compound (G) | | 1.4:1 | | 1:1 | | 1:1 | | 1.17:1 | | 1.17:1 | | 1.17:1 | | 1.25:1 | | 0.15:1 | | - | |
| 25°C for 3 mos. | | Initial 3 mos. | | Initial 3 mos. | | Initial 3 mos. | | Initial 3 mos. | | Initial 3 mos. | | Initial 3 mos. | | Initial 3 mos. | | Initial 3 mos. | | Initial 3 mos. | |
| | I-Menthol residual rate (%) | 101.1 | 100.4 | 101.1 | 100.0 | 101.2 | 100.1 | 100.7 | 99.0 | 100.6 | 100.1 | 101.2 | 100.2 | - | - | - | - | 96.9 | 97.8 |
| | Light transmittance (%) | 95.8 | 95.6 | 95.5 | 95.3 | 95.2 | 94.9 | 95.8 | 95.6 | 95.5 | 95.2 | 96.4 | 96.4 | 95.5 | - | 93.8 | - | 96.6 | 95.8 |
| | pH | 5.00 | 4.99 | 4.68 | 4.62 | 4.68 | 4.62 | 4.94 | 4.88 | 5.02 | 5.03 | 5.83 | 5.80 | 4.98 | - | 4.80 | - | 5.05 | 5.02 |
| | Average particle size (nm) | 17.0 | 17.0 | 16.0 | 18.0 | 17.0 | 18.0 | 17.0 | 18.0 | 17.0 | 18.0 | 16.0 | 17.0 | 17.0 | - | 22.0 | - | 18.1 | 18.3 |
| | Appearance | trans. liq. | trans. liq. | trans. liq. | trans. liq. | trans. liq. | trans. liq. | trans. liq. | trans. liq. | trans. liq. | trans. liq. | trans. liq. | trans. liq. | trans. liq | - | trans. liq. | - | trans. liq. | trans. liq. |
| 4°C for 3 mos. | | Initial | 3 mos. | Initial | 3 mos. | Initial | 3 mos. | Initial | 3 mos. | Initial | 3 mos. | Initial | 3 mos. | Initial | 3 mos. | Initial | 3 mos. | Initial | 3 mos. |
| | I-Menthol residual rate (%) | 101.1 | 100.9 | 101.1 | 101.5 | 101.2 | 101.0 | 100.7 | 100.5 | 100.6 | 100.6 | 101.2 | 100.9 | - | - | - | - | 96.9 | 97.8 |
| | Light transmittance (%) | 95.8 | 95.8 | 95.5 | 95.5 | 95.2 | 95.3 | 95.8 | 95.8 | 95.5 | 95.6 | 96.4 | 96.5 | 95.5 | - | 93.8 | - | 96.6 | 96.1 |
| | pH | 5.00 | 5.00 | 4.68 | 4.69 | 4.68 | 4.70 | 4.94 | 4.94 | 5.02 | 5.03 | 5.83 | 5.85 | 4.98 | - | 4.80 | - | 5.05 | 5.03 |
| | Average particle size (nm) | 17.0 | 17.0 | 16.0 | 17.0 | 17.0 | 17.0 | 17.0 | 17.0 | 17.0 | 17.0 | 16.0 | 16.0 | 17.0 | - | 22.0 | - | 18.1 | 18.0 |
| | Appearance | trans. liq. | trans. liq. | trans. liq. | trans. liq. | trans. liq. | trans. liq. | trans. liq. | trans. liq. | trans. liq. | trans. liq. | trans. liq. | trans. liq. | trans. liq. | - | trans. liq. | precipitates | trans. liq. | crystals |

| | | | | | | | | | | | | | | | | | | | |
|---|---|---|---|---|---|---|---|---|---|---|---|---|---|---|---|---|---|---|---|
| The symbol "-" in the table indicates that data in question was unmeasured. | | | | | | | | | | | | | | | | | | | |

The results in Tables 5 and 6 demonstrate that the emulsion compositions according to the examples each had a transparent appearance after storage at 4°C and 25°C for 3 months, and, over the entire period, had a 1-menthol residual rate (%) of not less than 97%, a light transmittance (%) of not less than 90%, a pH of about 5 to 6 without significant variation, and a stable average particle size (nm) of about 20 nm. In contrast, the emulsion composition according to Comparative Example 2-1 was prepared according to the formulation having a weight ratio of the component (b2) to the component (G) of lower than the lower limit, where the component (b2) is a medium-chain fatty acid and/or a salt thereof, and the component (G) is a benzoic acid (benzoic acid or its derivative). This sample presented a transparent appearance at the beginning of the experiment, but included precipitates (white foreign substance) after storage at 4°C for 3 months, and had poor storage stability. The precipitates (white foreign substance) were qualitatively analyzed by mass spectrometry (MS) and found to include a mixture of a diester and a triester derived from the sucrose fatty acid ester. The emulsion composition according to Comparative Example 2-2 was prepared according to the formulation devoid of the component (G). This sample presented a transparent appearance at the beginning of the experiment, but included crystals after storage at 4°C for 3 months and had poor storage stability. These data probably indicate that the control of the weight ratio of content of the component (b2) to that of the component (G) within the specific range maintains the good stability of the preparations, where the component (b2) is a medium-chain fatty acid and/or a salt thereof, and the component (G) is a benzoic acid.

### Experiment 2-6: Long-term Storage Stability

The emulsion compositions according to the examples and the comparative examples were evaluated for long-term storage stability. Each emulsion composition after storage for 6 months, 9 months, and 12 months from the beginning of the experiment underwent the five experiments. The results are given in Tables 7 to 10.

### [Table 7]

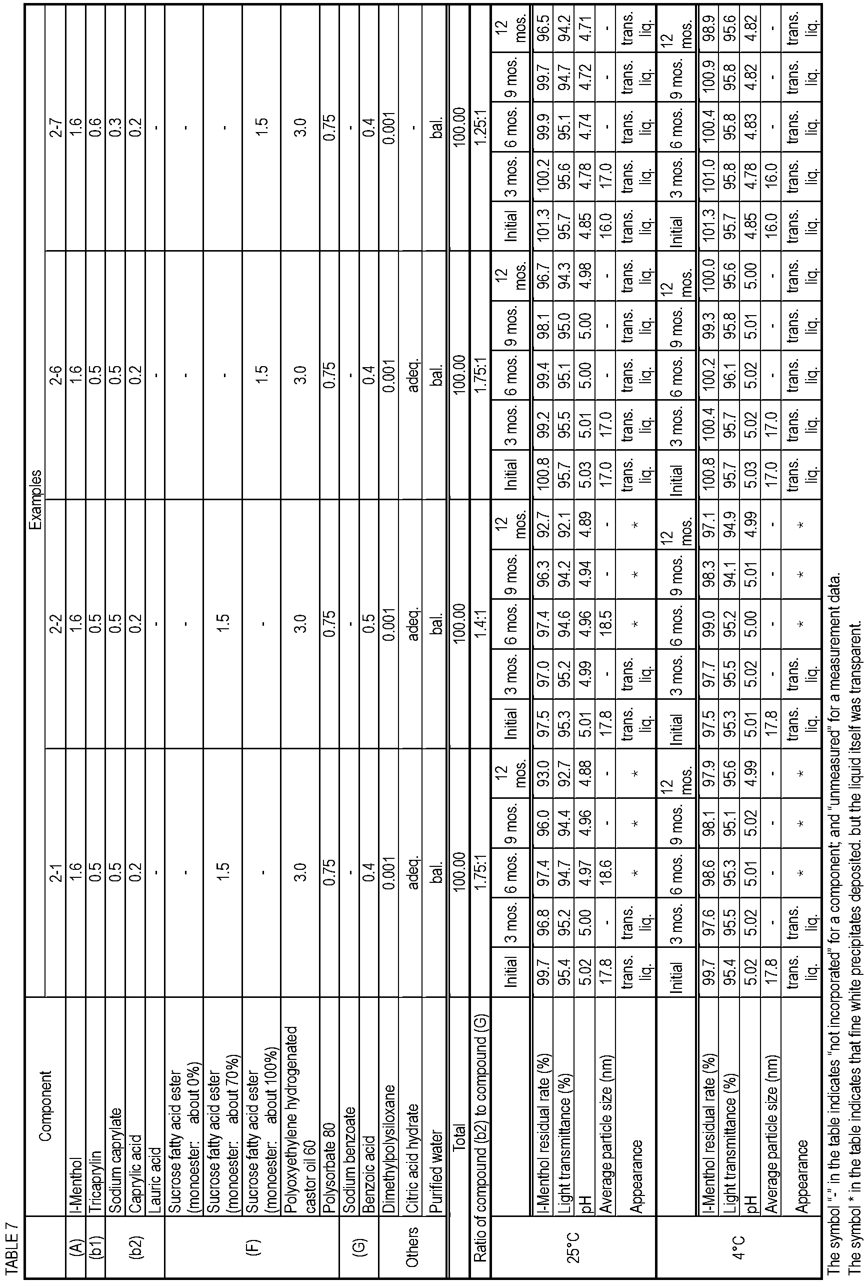

### [Table 8]

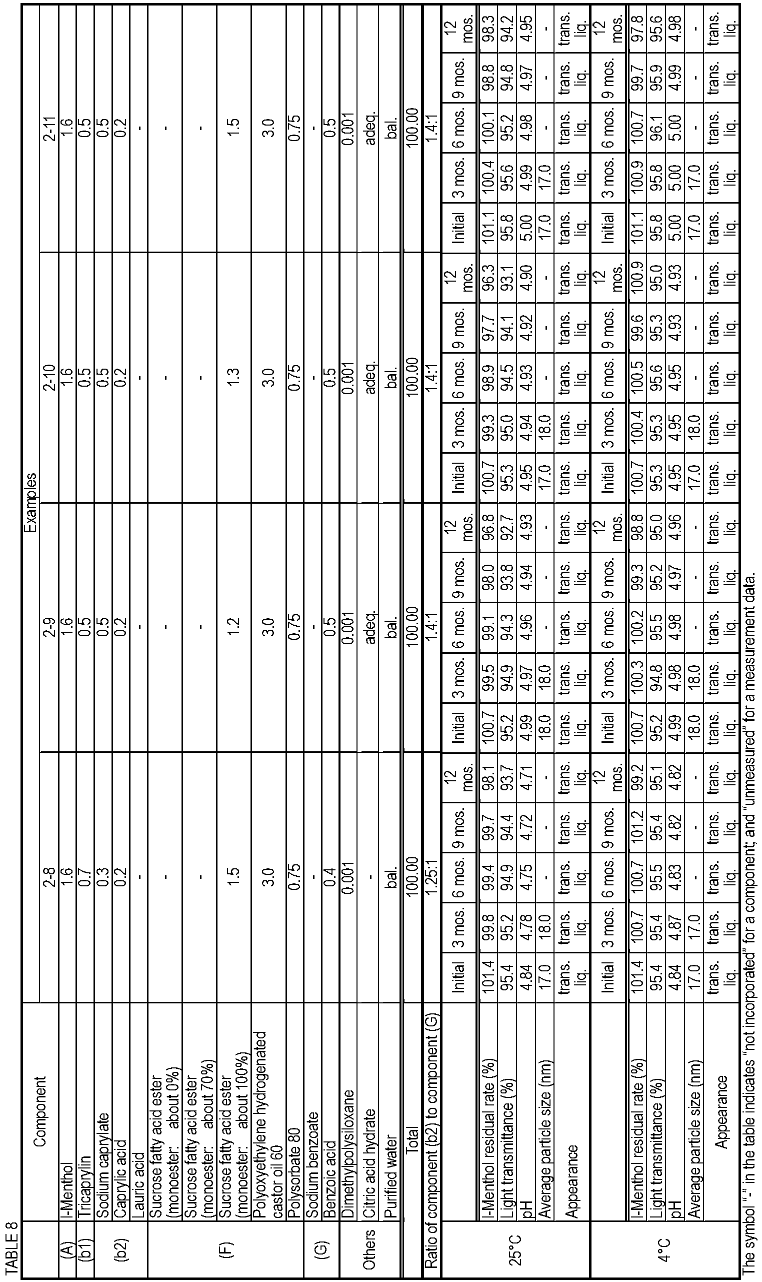

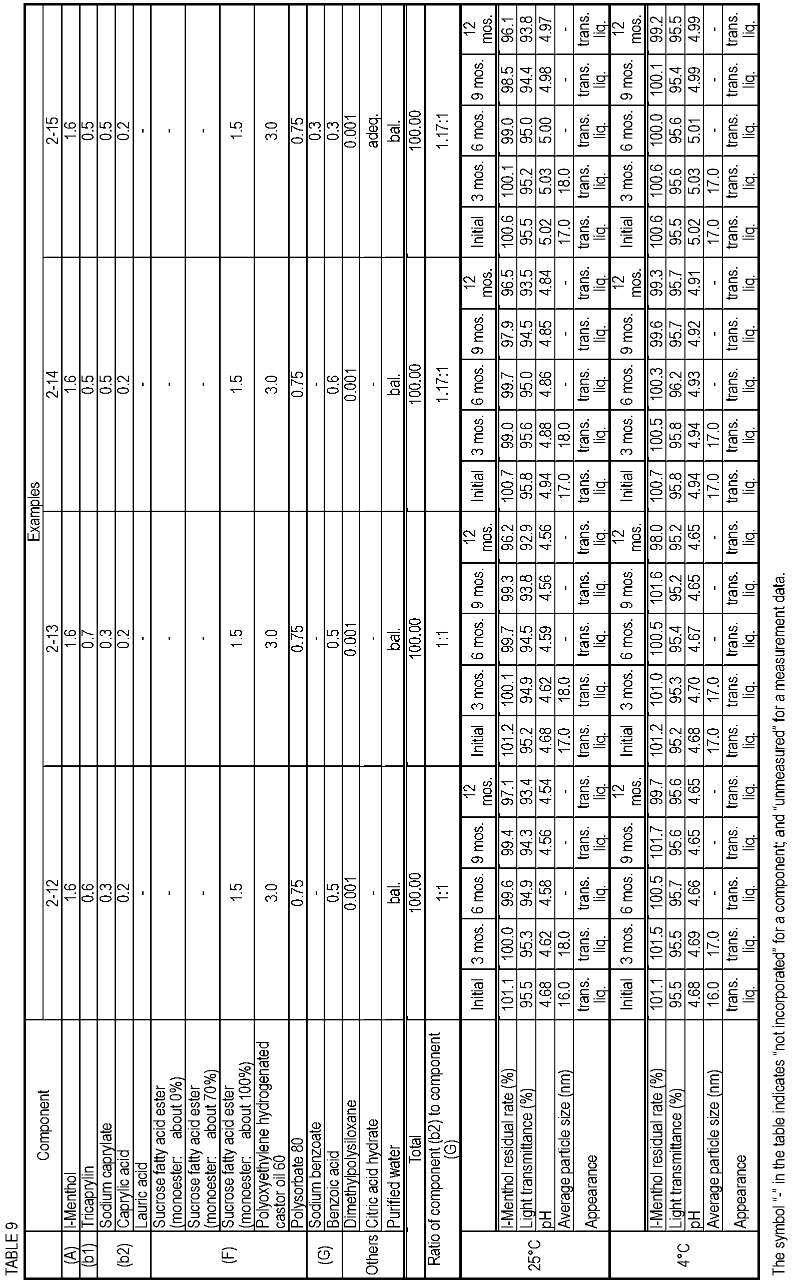

### [Table 10]

**TABLE 10**

| | Component | Example | | | | | Comparative Example | | | | |
|---|---|---|---|---|---|---|---|---|---|---|---|
| | | 2-16 | | | | | 2-2 | | | | |
| (A) | I-Menthol | 1.6 | | | | | 1.6 | | | | |
| (b1) | Tricaprylin | 0.5 | | | | | 0.5 | | | | |
| (b2) | Sodium caprylate | 0.5 | | | | | 0.5 | | | | |
| | Caprylic acid | 0.2 | | | | | - | | | | |
| | Lauric acid | - | | | | | - | | | | |
| (F) | Sucrose fatty acid ester (monoester: about 0%) | - | | | | | - | | | | |
| | Sucrose fatty acid ester (monoester: about 70%) | - | | | | | 1.5 | | | | |
| | Sucrose fatty acid ester (monoester: about 100%) | 1.5 | | | | | - | | | | |
| | Polyoxyethylene hydrogenated castor oil 60 | 3.0 | | | | | 3.0 | | | | |
| | Polysorbate 80 | 0.75 | | | | | 0.75 | | | | |
| (G) | Sodium benzoate | 0.3 | | | | | - | | | | |
| | Benzoic acid | 0.3 | | | | | - | | | | |
| Others | Dimethylpolysiloxane | 0.001 | | | | | 0.001 | | | | |
| | Citric acid hydrate | - | | | | | adeq. | | | | |
| | Purified water | bal. | | | | | bal. | | | | |
| Total | | 100.00 | | | | | 100.00 | | | | |
| Ratio of component (b2) to component (G) | | 1.17:1 | | | | | - | | | | |
| 25°C | | Initial | 3 mos. | 6 mos. | 9 mos. | 12 mos. | Initial | 3 mos. | 6 mos. | 9 mos. | 12 mos. |
| | I-Menthol residual rate (%) | 101.2 | 100.2 | 99.3 | 99.6 | 98.5 | 96.9 | 97.8 | 96.5 | 97.1 | 95.7 |
| | Light transmittance (%) | 96.4 | 96.4 | 96.1 | 95.8 | 95.3 | 96.6 | 95.8 | 95.0 | 95.0 | 95.3 |
| | pH | 5.83 | 5.80 | 5.72 | 5.71 | 5.69 | 5.05 | 5.02 | 5.03 | 5.03 | 5.01 |
| | Average particle size (nm) | 16.0 | 17.0 | - | - | - | 18.1 | 18.3 | - | - | - |
| | Appearance | trans. liq. | trans. liq. | trans. liq. | trans. liq. | trans. liq. | trans. liq. | trans. liq. | * | * | * |
| 4°C | | Initial | 3 mos. | 6 mos. | 9 mos. | 12 mos. | Initial | 3 mos. | 6 mos. | 9 mos. | 12 mos. |
| | I-Menthol residual rate (%) | 101.2 | 100.9 | 100.4 | 100.6 | 99.9 | 96.9 | 97.8 | 97.0 | 97.8 | 97.9 |
| | Light transmittance (%) | 96.4 | 96.5 | 96.5 | 96.5 | 96.5 | 96.6 | 96.1 | 95.8 | 95.5 | 95.5 |
| | pH | 5.83 | 5.85 | 5.81 | 5.80 | 5.79 | 5.05 | 5.03 | 5.04 | 5.05 | 5.05 |
| | Average particle size (nm) | 16.0 | 16.0 | - | - | - | 18.1 | 18.0 | - | - | - |
| | Appearance | trans. liq. | trans. liq. | trans. liq. | trans. liq. | trans. liq. | trans. liq. | crystal | crystal | crystal | crystal |

| | | | | | | | | | | | |
|---|---|---|---|---|---|---|---|---|---|---|---|
| The symbol "-" in the table indicates "not incorporated" for a component; and "unmeasured" for a measurement data. The symbol * in the table indicates that fine white precipitates deposited. but the liquid itself was transparent. | | | | | | | | | | | |

The data in Tables 7 to 10 demonstrate that the emulsion compositions according to Example 2-1, 2-2, and 2-6 to 2-16 were stable after storage at 4°C and 25°C for 12 months. These results indicate that the emulsion compositions according to the examples have excellent long-term storage stability. The emulsion compositions according to Examples 2-1 and 2-2 included very fine, white precipitates, but were liquids having a transparent appearance after storage at 4°C or 25°C for 6 months. These emulsion compositions could be evaluated as being practically usable without problems. This is because the gastrointestinal smooth muscle contraction inhibitors according to the second embodiment of the present invention are to be administered to smooth muscles of a digestive organ (digestive tract) such as intestine, and the emulsion compositions, when sprayed in such an application, are approximately free from the possibility of masking an affected part.

In contrast, the emulsion composition according to Comparative Example 2-2 included fine white precipitates, although the liquid itself was transparent, after storage at 25°C for 6 months and thereafter. In addition, this sample included crystals after storage at 4°C for 3 months and thereafter and was found to have poor storage stability. The crystals observed after storage at 4°C for 3 months and thereafter are assumed to be needle crystals of 1-menthol (component (A)).

As a summary of the above description, the configurations according to embodiments of the present invention, as well as variations (other embodiments) thereof, will be listed below as appendices.
(1) A pharmaceutical composition including components (A), (B), (C), (D), and (E) and having a cloud point of 30°C to 75°C (more preferably 35°C to 70°C, and still more preferably 35°C to 65°C), where the components are as follows:
   Component (A): l-menthol;
   Component (B): at least one selected from the group consisting of a medium-chain fatty acid triacylglycerol, a medium-chain fatty acid, and a salt of medium-chain fatty acid;
   Component (C): a nonionic surfactant having an HLB of not less than 10;
   Component (D): a polyhydric alcohol having not less than three hydroxy groups per molecule and/or a poly(oxyalkylene) glycol; and
   Component (E): water.
(2) The pharmaceutical composition according to (1), wherein the weight ratio of content of the component (C) to that of the component (D) [component (C) / component (D)] is 0.2 to 5.0 (more preferably 0.4 to 4.0, and still more preferably 0.6 to 3.0).
(3) The pharmaceutical composition according to one of (1) and (2), wherein the component (C) is at least one selected from the group consisting of a sucrose fatty acid ester, a polyoxyethylene hydrogenated castor oil, and a polysorbate.
(4) The pharmaceutical composition according to any one of (1) to (3), wherein the component (C) has a content of the sucrose fatty acid esters of 0 to 0.1 weight % (preferably not more than 0.01 weight %, and more preferably not more than 0.001 weight %) of the total amount of the component (C).
(5) The pharmaceutical composition according to any one of (1) to (4), wherein the component (D) is at least one selected from the group consisting of glycerol, D-sorbitol, D-mannitol, and macrogol 400.
(6) The pharmaceutical composition according to (5), wherein the component (D) is D-sorbitol and/or D-mannitol.
(7) The pharmaceutical composition according to any one of (1) to (6), which has a pH of 3 to 8.
(8) The pharmaceutical composition according to any one of (1) to (7),
   wherein:
   the content of component (A) is 0.01 to 5 weight %,
   the content of component (B) is 0.1 to 10 weight %,
   the content of component (C) is 3 to 15 weight %, and
   the content of component (D) is 3 to 20 weight %.
(9) The pharmaceutical composition according to (8), wherein the content of component (A) is 0.1 to 4 weight % (preferably more than 0.8 weight % but not more than 3 weight %, and more preferably 1 to 2 weight %).
(10) The pharmaceutical composition according to one of (8) and (9), wherein the content of component (B) is 0.4 to 8 weight % (and preferably 0.5 to 7 weight %).
(11) The pharmaceutical composition according to any one of (8) to (10), wherein the content of component (C) is 4 to 13 weight % (and preferably 5 to 12 weight %).
(12) The pharmaceutical composition according to any one of (8) to (11), wherein the content of component (D) is 5 to 18 weight % (and preferably 8 to 15 weight %).
(13) The pharmaceutical composition according to any one of (1) to (12), wherein the pharmaceutical composition is an emulsion, and wherein paticles in the emulsion have an average particle size of less than 100 nm (preferably not more than 70 nm, and more preferably not more than 50 nm).
(14) The pharmaceutical composition according to any one of (1) to (13), which has a light transmittance of not less than 50% (and preferably not less tahn 70%).
(15) The pharmaceutical composition according to any one of (1) to (14), which is a gastrointestinal smooth muscle contraction inhibitor.
(16) A method for stabilizing a pharmaceutical composition including 1-menthol, at least one selected from the group consisting of a medium-chain fatty acid triacylglycerol, a medium-chain fatty acid, and a salt of medium-chain fatty acid, a nonionic surfactant having an HLB of not less than 10, and water, the method including combining the pharmaceutical composition with a polyhydric alcohol having not less than three hydroxy groups per molecule and/or a poly(oxyalkylene) glycol, adjusting the content each of the components, making the cloud point of the pharmaceutical composition 30°C to 75°C, and whereby stabilizing the emulsion state of the pharmaceutical composition.
(17) A method for evaluating storage stability of a pharmaceutical composition including components (A), (B), (C), and water, the method including measuring the cloud point of the pharmaceutical composition, determining a difference between the cloud point and a storage temperature of the pharmaceutical composition, and evaluating the storage stability of the pharmaceutical composition by taking the difference as an index, where the components (A), (B), and (C) are as follows:
   Component (A): l-menthol;
   Component (B): at least one selected from the group consisting of a medium-chain fatty acid triacylglycerol, a medium-chain fatty acid, and a salt of medium-chain fatty acid; and
   Component (C): a nonionic surfactant having an HLB of not less than 10.
(18) A gastrointestinal smooth muscle contraction inhibitor containing components (A), (b1), (b2), (F), and (G),
   wherein:
   the content of component (A) is 0.01 to 5 weight %,
   the content of component (b1) is 0.1 to 10 times as much by weight as that of the component (A),
   the content of component (b2) is 0.1 to 10 times as much by weight as that of the component (A),
   the content of component (F) is 0.1 to 10 times as much by weight as the total amount of the component (A), the component (b1), and the component (b2),
   the content of component (G) is 0.01 to 2.0 weight % as benzoic acid, and
   the weight ratio of the component (b2) to the component (G) [component (b2) / component (G)] is from 1/5 to 10/1, wherein the components (A), (b1), (b2), (F), and (G) are as follows:
      Component (A): l-menthol;
      Component (b1): a medium-chain fatty acid triacylglycerol;
      Component (b2): a medium-chain fatty acid and/or a salt thereof;
      Component (F): at least one selected from the group consisting of a sucrose fatty acid ester, a polyoxyethylene hydrogenated castor oil, and a polysorbate; and
      Component (G): at least one selected from the group consisting of benzoic acid, p-hydroxybenzoic acid, and a salt or an alkyl ester thereof.
(19) The gastrointestinal smooth muscle contraction inhibitor according to (18), wherein the content of component (A) is more than 0.8 weight % but not more than 3 weight % (and preferably 1 to not more than 2 weight %).
(20) The gastrointestinal smooth muscle contraction inhibitor according to one of (18) and (19), wherein the content of component (b1) is 0.2 to 5 times as much by weight as that of the component (A).
(21) The gastrointestinal smooth muscle contraction inhibitor according to any one of (18) to (20), wherein the content of component (b1) is 0.3 to 1 weight % (preferably 0.4 to 0.9 weight %, and more preferably 0.5 to 0.7 weight %).
(22) The gastrointestinal smooth muscle contraction inhibitor according to any one of (18) to (21), wherein the content of component (b2) is 0.2 to 5 times as much by weight as that of the component (A).
(23) The gastrointestinal smooth muscle contraction inhibitor according to any one of (18) to (22), wherein the content of component (b2) is 0.2 to 1 weight % (preferably 0.4 to 0.9 weight %, and more preferably 0.5 to 0.7 weight %).
(24) The gastrointestinal smooth muscle contraction inhibitor according to any one of (18) to (23), wherein the content of component (F) is 0.5 to 5 times as much by weight as the total amount of the component (A), the component (b1), and the component (b2).
(25) The gastrointestinal smooth muscle contraction inhibitor according to any one of (18) to (24), wherein the content of component (F) is 0.1 to 20 weight % (and preferably 0.5 to 15 weight %).
(26) The gastrointestinal smooth muscle contraction inhibitor according to any one of (18) to (25), wherein the component (G) is present in a content as benzoic acid of 0.05 to 1.5 weight % (and preferably 0.3 to 0.9 weight %).
(27) The gastrointestinal smooth muscle contraction inhibitor according to any one of (18) to (26), wherein the weight ratio of the component (b2) to the component (G) [component (b2) / component (G)] is from 1/5 to 5/1 (preferably from 1/3 to 3/1, and more preferably from 1/2 to 2/1) .
(28) The gastrointestinal smooth muscle contraction inhibitor according to any one of (18) to (27), wherein the inhibitor includes, as the component (F), a sucrose fatty acid ester, a polyoxyethylene hydrogenated castor oil, and a polysorbate.
(29) The gastrointestinal smooth muscle contraction inhibitor according to any one of (18) to (28), wherein the inhibitor includes, as the component (F), a sucrose fatty acid ester, and wherein the sucrose fatty acid ester includes a sucrose fatty acid monoester in a proportion of not less than 55 weight % (preferably not less than 60 weight %, and more preferably not less than 65 weight %) of the total amount of the sucrose fatty acid ester.
(30) The gastrointestinal smooth muscle contraction inhibitor according to any one of (18) to (29), wherein the component (b1) is a triacylglycerol of a C₆-C₁₂ fatty acid.
(31) The gastrointestinal smooth muscle contraction inhibitor according to any one of (18) to (30), wherein the component (b1) is tricaprylin.
(32) The gastrointestinal smooth muscle contraction inhibitor according to any one of (18) to (31), wherein the component (b2) is a C₆-C₁₂ fatty acid and/or a salt thereof.
(33) The gastrointestinal smooth muscle contraction inhibitor according to any one of (18) to (32), wherein the inhibitor includes, as the component (b2), lauric acid, caprylic acid, and sodium caprylate.
(34) The gastrointestinal smooth muscle contraction inhibitor according to any one of (18) to (33), wherein the smooth muscle contraction inhibitor is an emulsion, and wherein particles in the emulsion have an average particle size of less than 100 nm (preferably not more than 70 nm, and more preferably not more than 50 nm).
(35) The gastrointestinal smooth muscle contraction inhibitor according to any one of (18) to (34), which has a light transmittance of not less than 50% (and preferably not less than 70%).
(36) A method for inhibiting gastrointestinal smooth muscle contraction, the method including administering to a mammal the pharmaceutical composition according to any one of (1) to (14).
(37) Use of the pharmaceutical composition according to any one of (1) to (14) for inhibiting gastrointestinal smooth muscle contraction.
(38) A method for inhibiting gastrointestinal smooth muscle contraction, the method including administering to a mammal a pharmaceutical composition,
   wherein:
   the pharmaceutical composition containing components (A), (b1), (b2), (F), and (G) as follows,
   the content of component (A) is 0.01 to 5 weight %,
   the content of component (b1) is 0.1 to 10 times as much by weight as that of the component (A),
   the content of component (b2) is 0.1 to 10 times as much by weight as that of the component (A),
   the content of component (F) is 0.1 to 10 times as much by weight as the total amount of the component (A), the component (b1), and the component (b2),
   the content of component (G) is 0.01 to 2.0 weight % as benzoic acid, and
   the weight ratio of the component (b2) to the component (G) [component (b2) / component (G)] is from 1/5 to 10/1, where the components (A), (b1), (b2), (F), and (G) are as follows:
      Component (A): l-menthol;
      Component (b1): a medium-chain fatty acid triacylglycerol;
      Component (b2): a medium-chain fatty acid and/or a salt thereof;
      Component (F): at least one selected from the group consisting of a sucrose fatty acid ester, a polyoxyethylene hydrogenated castor oil, and a polysorbate; and
      Component (G): at least one selected from the group consisting of benzoic acid, p-hydroxybenzoic acid, and a salt or an alkyl ester thereof.
(39) The method according to (38) for inhibiting gastrointestinal smooth muscle contraction, wherein the content of component (A) in a content of from more than 0.8 weight % but not more than 3 weight % (and preferably 1 to 2 weight %) relative to the total amount of the pharmaceutical composition.
(40) The method according to one of (38) and (39) for inhibiting gastrointestinal smooth muscle contraction, wherein the content of component (b1) is 0.2 to 5 times as much by weight as that of the component (A).
(41) The method according to any one of (38) to (40) for inhibiting gastrointestinal smooth muscle contraction, wherein the content of component (b1) is 0.3 to 1 weight % (preferably 0.4 to 0.9 weight %, and more preferably 0.5 to 0.7 weight %).
(42) The method according to any one of (38) to (41) for inhibiting gastrointestinal smooth muscle contraction, wherein the content of component (b2) is 0.2 to 5 times as much by weight as that of the component (A).
(43) The method according to any one of (38) to (42) for inhibiting gastrointestinal smooth muscle contraction, wherein the content of component (b2) is 0.2 to 1 weight % (preferably 0.4 to 0.9 weight %, and more preferably 0.5 to 0.7 weight %) relative to the total amount of the pharmaceutical composition.
(44) The method according to any one of (38) to (43) for inhibiting gastrointestinal smooth muscle contraction, wherein the content of component (F) is 0.5 to 5 times as much by weight as the total amount of the component (A), the component (b1), and the component (b2).
(45) The method according to any one of (38) to (44) for inhibiting gastrointestinal smooth muscle contraction, wherein the content of component (F) in a content of 0.1 to 20 weight % (and preferably 0.5 to 15 weight %) relative to the total amount of the pharmaceutical composition.
(46) The method according to any one of (38) to (45) for inhibiting gastrointestinal smooth muscle contraction, wherein the pharmaceutical composition contains the component (G) in a content as benzoic acid of 0.05 to 1.5 weight % (and preferably 0.3 to 0.9 weight %).
(47) The method according to any one of (38) to (46) for inhibiting gastrointestinal smooth muscle contraction, wherein the weight ratio of the component (b2) to the component (G) [component (b2) / component (G)] is from 1/5 to 5/1 (preferably from 1/3 to 3/1, and more preferably from 1/2 to 2/1).
(48) The method according to any one of (38) to (47) for inhibiting gastrointestinal smooth muscle contraction, wherein the pharmaceutical composition includes, as the component (F), a sucrose fatty acid ester, a polyoxyethylene hydrogenated castor oil, and a polysorbate.
(49) The method according to any one of (38) to (48) for inhibiting gastrointestinal smooth muscle contraction, wherein in the pharmaceutical composition includes, the component (F), a sucrose fatty acid ester, and the sucrose fatty acid ester includes a sucrose fatty acid monoester in a content of not less than 55 weight % (preferably not less than 60 weight %, and more preferably not less than 65 weight %) of the total amount of the sucrose fatty acid ester.
(50) The method according to any one of (38) to (49) for inhibiting gastrointestinal smooth muscle contraction, wherein the component (b1) is a triacylglycerol of a C₆-C₁₂ fatty acid.
(51) The method according to any one of (38) to (50) for inhibiting gastrointestinal smooth muscle contraction, wherein the pharmaceutical composition includes tricaprylin as the component (b1).
(52) The method according to any one of (38) to (51) for inhibiting gastrointestinal smooth muscle contraction, wherein the pharmaceutical composition includes a C₆-C₁₂ fatty acid and/or a salt thereof as the component (b2).
(53) The method according to any one of (38) to (52) for inhibiting gastrointestinal smooth muscle contraction, wherein the pharmaceutical composition includes lauric acid, caprylic acid, and sodium caprylate as the component (b2).
(54) The method according to any one of (38) to (53) for inhibiting gastrointestinal smooth muscle contraction, wherein the pharmaceutical composition is an emulsion, and wherein particles in the emulsion have an average particle size of less than 100 nm (preferably not more than 70 nm, and more preferably not more than 50 nm).
(55) The method according to any one of (38) to (54) for inhibiting gastrointestinal smooth muscle contraction, wherein the pharmaceutical composition has a light transmittance of not less than 50% (and preferably not less than 70%).
(56) Use of a pharmaceutical composition for inhibiting gastrointestinal smooth muscle contraction,
   wherein:
   the pharmaceutical composition contains components (A), (b1), (b2), (F), and (G),
   the content of component (A) is 0.01 to 5 weight %,
   the content of component (b1) is 0.1 to 10 times as much by weight as that of the component (A),
   the content of component (b2) is 0.1 to 10 times as much by weight as that of the component (A),
   the content of component (F) is 0.1 to 10 times as much by weight as the total amount of the component (A), the component (b1), and the component (b2),
   the content of component (G) is 0.01 to 2.0 weight % as benzonic acid, and
   the weight ratio of the component (b2) to the component (G) [component (b2) / component (G)] is from 1/5 to 10/1,
   where the components (A), (b1), (b2), (F), and (G) are as follows:
      Component (A): l-menthol;
      Component (b1): a medium-chain fatty acid triacylglycerol;
      Component (b2): a medium-chain fatty acid and/or a salt thereof;
      Component (F): at least one selected from the group consisting of a sucrose fatty acid ester, a polyoxyethylene hydrogenated castor oil, and a polysorbate; and
      Component (G): at least one selected from the group consisting of benzoic acid, p-hydroxybenzoic acid, and a salt or an alkyl ester thereof.
(57) The use according to (56) of a pharmaceutical composition for inhibiting gastrointestinal smooth muscle contraction, wherein the content of component (A) is more than 0.8 weight % but not more than 3 weight % (and preferably 1 to not more than 2 weight %) relative to the total amount of the pharmaceutical composition.
(58) The use according to one of (56) and (57) of a pharmaceutical composition for inhibiting gastrointestinal smooth muscle contraction, wherein the content of component (b1) is 0.2 to 5 times as much by weight as that of the component (A).
(59) The use according to any one of (56) to (58) of a pharmaceutical composition for inhibiting gastrointestinal smooth muscle contraction, wherein the content of component (b1) is 0.3 to 1 weight % (preferably 0.4 to 0.9 weight %, and more preferably 0.5 to 0.7 weight %).
(60) The use according to any one of (56) to (59) of a pharmaceutical composition for inhibiting gastrointestinal smooth muscle contraction, wherein the content of component (b2) is 0.2 to 5 times as much by weight as that of the component (A).
(61) The use according to any one of (56) to (60) of a pharmaceutical composition for inhibiting gastrointestinal smooth muscle contraction, wherein the content of component (b2) is 0.2 to 1 weight % (preferably 0.4 to 0.9 weight %, and more preferably 0.5 to 0.7 weight %) relative to the total amount of the pharmaceutical composition.
(62) The use according to any one of (56) to (61) of a pharmaceutical composition for inhibiting gastrointestinal smooth muscle contraction, wherein the content of component (F) is 0.5 to 5 times as much by weight as the total amount of the component (A), the component (b1), and the component (b2) .
(63) The method according to any one of (56) to (62) for inhibiting gastrointestinal smooth muscle contraction, wherein the content of component (F) is 0.1 to 20 weight % (and preferably 0.5 to 15 weight %) relative to the total amount of the pharmaceutical composition.
(64) The use according to any one of (56) to (63) of a pharmaceutical composition for inhibiting gastrointestinal smooth muscle contraction, wherein the pharmaceutical composition contains the component (G) in a content as benzoic acid of 0.05 to 1.5 weight % (and preferably 0.3 to 0.9 weight %).
(65) The use according to any one of (56) to (64) of a pharmaceutical composition for inhibiting gastrointestinal smooth muscle contraction, wherein the weight ratio of the component (b2) to the component (G) [component (b2) / component (G)] is from 1/5 to 5/1 (preferably from 1/3 to 3/1, and more preferably from 1/2 to 2/1).
(66) The use according to any one of (56) to (65) of a pharmaceutical composition for inhibiting gastrointestinal smooth muscle contraction, wherein the pharmaceutical composition includes a sucrose fatty acid ester, a polyoxyethylene hydrogenated castor oil, and a polysorbate as the component (F).
(67) The use according to any one of (56) to (66) of a pharmaceutical composition for inhibiting gastrointestinal smooth muscle contraction, wherein the pharmaceutical composition includes, as the component (F), a sucrose fatty acid ester, and the sucrose fatty acid ester includes a sucrose fatty acid monoester in a proportion of not less than 55 weight % (preferably not less than 60 weight %, and more preferably not less than 65 weight %) of the total amount of the sucrose fatty acid ester.
(68) The use according to any one of (56) to (67) of a pharmaceutical composition for inhibiting gastrointestinal smooth muscle contraction, wherein the pharmaceutical composition includes a triacylglycerol of a C₆-C₁₂ fatty acid as the component (b1).
(69) The use according to any one of (56) to (68) of a pharmaceutical composition for inhibiting gastrointestinal smooth muscle contraction, wherein the pharmaceutical composition includes tricaprylin as the component (b1).
(70) The use according to any one of (56) to (69) of a pharmaceutical composition for inhibiting gastrointestinal smooth muscle contraction, wherein the pharmaceutical composition includes a C₆-C₁₂ fatty acid and/or a salt thereof as the component (b2).
(71) The use according to any one of (56) to (70) of a pharmaceutical composition for inhibiting gastrointestinal smooth muscle contraction, wherein the pharmaceutical composition includes lauric acid, caprylic acid, and sodium caprylate as the component (b2).
(72) The use according to any one of (56) to (71) of a pharmaceutical composition for inhibiting gastrointestinal smooth muscle contraction, wherein the pharmaceutical composition is an emulsion, and wherein particles in the emulsion have an average particle size of less than 100 nm (preferably not more than 70 nm, and more preferably not more than 50 nm).
(73) The use according to any one of (56) to (72) of a pharmaceutical composition for inhibiting gastrointestinal smooth muscle contraction, wherein the pharmaceutical composition has a light transmittance of not less than 50% (and preferably not less than 70%).

### Industrial Applicability

(0229) The pharmaceutical composition according to the first embodiment of the present invention has excellent storage stability even when containing 1-menthol in a high concentration. The method according to the first embodiment of the present invention for stabilizing a pharmaceutical composition can give a preparation that is highly stable when existing as an emulsion. The method according to the first embodiment of the present invention for evaluating the storage stability of a pharmaceutical composition enables easy designing of a pharmaceutical composition that is highly stable with time. The gastrointestinal smooth muscle contraction inhibitor according to the second embodiment of the present invention is highly stable at low temperatures even when containing 1-menthol in a high concentration.

## Claims

1. A pharmaceutical composition having a cloud point of 30°C to 75°C and comprising components (A), (B), (C), (D) and (E) as follows:
Component (A): l-menthol;
Component (B): at least one selected from the group consisting of:
a medium-chain fatty acid triacylglycerol;
a medium-chain fatty acid; and
a salt of a medium-chain fatty acid;
Component (C): a nonionic surfactant having a hydrophile-lipophile balance (HLB) of not less than 10;
Component (D): a polyhydric alcohol having not less than three hydroxy groups per molecule and/or a poly(oxyalkylene) glycol; and
Component (E): water.

2. The pharmaceutical composition according to claim 1,
wherein the weight ratio of the content of component (C) to that of the component (D) [component (C)/component (D)] is from 0.2 to 5.0.

3. The pharmaceutical composition according to one of claims 1 and 2,
wherein the component (C) is at least one selected from the group consisting of:
a sucrose fatty acid ester;
a polyoxyethylene hydrogenated castor oil; and
a polysorbate.

4. The pharmaceutical composition according to any one of claims 1 to 3,
wherein the component (C) has a content of the sucrose fatty acid esters of not more than 0.1 weight %.

5. The pharmaceutical composition according to any one of claims 1 to 4,
wherein the component (D) is at least one selected from the group consisting of:
glycerol;
D-sorbitol;
D-mannitol; and
macrogol 400.

6. The pharmaceutical composition according to any one of claims 1 to 5,
which has a pH of 3 to 8.

7. The pharmaceutical composition according to any one of claims 1 to 6,
wherein:
the content of component (A) is 0.01 to 5 weight %,
the content of component (B) is 0.1 to 10 weight %,
the content of component (C) is 3 to 15 weight %, and
the content of component (D) is 3 to 20 weight %.

8. The pharmaceutical composition according to any one of claims 1 to 7,
wherein the pharmaceutical composition is an emulsion, and
wherein particles in the emulsion have an average particle size of less than 100 nm.

9. The pharmaceutical composition according to any one of claims 1 to 8,
which has a light transmittance of not less than 50%.

10. The pharmaceutical composition according to any one of claims 1 to 9,
which is a gastrointestinal smooth muscle contraction inhibitor.

11. A method for stabilizing a pharmaceutical composition which comprises:
l-menthol;
at least one selected from the group consisting of:
a medium-chain fatty acid triacylglycerol;
a medium-chain fatty acid; and
a salt of medium-chain fatty acid;
a nonionic surfactant having a hydrophile-lipophile balance (HLB) of not less than 10; and
water,
the method comprising:
combining the pharmaceutical composition with a polyhydric alcohol having not less than three hydroxy groups per molecule and/or a poly(oxyalkylene) glycol;
adjusting the content each of the components; and
making the cloud point of the pharmaceutical composition 30°C to 75°C;
whereby stabilizing the emulsified state of the pharmaceutical composition.

12. A method for evaluating storage stability of a pharmaceutical composition, wherein the pharmaceutical composition comprises components (A), (B), (C), and water, the method comprising:
measuring a cloud point of the pharmaceutical composition;
determining a difference between the cloud point and a storage temperature of the pharmaceutical composition; and
evaluating the storage stability of the pharmaceutical composition by taking the difference as an index,
where the components (A), (B), and (C) are as follows:
Component (A): l-menthol;
Component (B): at least one selected from the group consisting of:
a medium-chain fatty acid triacylglycerol;
a medium-chain fatty acid; and
a salt of medium-chain fatty acid; and
Component (C): a nonionic surfactant having a hydrophile-lipophile balance (HLB) of not less than 10.

13. A gastrointestinal smooth muscle contraction inhibitor comprising components (A), (b1), (b2), (F), and (G), wherein:
the content of component (A) is 0.01 to 5 weight %,
the content of component (b1) is 0.1 to 10 times as much by weight as that of the component (A),
the content of component (b2) is 0.1 to 10 times as much by weight as that of the component (A),
the content of component (F) is 0.1 to 10 times as much by weight as the total amount of the component (A), the component (b1), and the component (b2),
the content of component (G) is 0.01 to 2.0 weight % as benzoic acid, and
the weight ratio of the component (b2) to the component (G) [component (b2) / component (G)] is from 1/5 to 10/1, wherein the components (A), (b1), (b2), (F), and (G) are as follows:
Component (A): l-menthol;
Component (b1): a medium-chain fatty acid triacylglycerol;
Component (b2): a medium-chain fatty acid and/or a salt thereof;
Component (F): at least one selected from the group consisting of:
a sucrose fatty acid ester;
a polyoxyethylene hydrogenated castor oil; and
a polysorbate; and
Component (G): at least one selected from the group consisting of:
benzoic acid;
p-hydroxybenzoic acid; and
a salt or an alkyl ester thereof.

14. The gastrointestinal smooth muscle contraction inhibitor according to claim 13,
wherein the content of component (A) is more than 0.8 weight % but not more than 3 weight %.

15. The gastrointestinal smooth muscle contraction inhibitor according to one of claims 13 and 14,
wherein the inhibitor comprises, as the component (F):
a sucrose fatty acid ester;
a polyoxyethylene hydrogenated castor oil; and
a polysorbate.

16. The gastrointestinal smooth muscle contraction inhibitor according to any one of claims 13 to 15,
which comprises, as the component (F), a sucrose fatty acid ester, and
wherein the sucrose fatty acid ester comprises a sucrose fatty acid monoester in a proportion of not less than 55 weight % of the total amount of the sucrose fatty acid ester.

17. The gastrointestinal smooth muscle contraction inhibitor according to any one of claims 13 to 16,
wherein the component (b1) is a triacylglycerol of a C₆-C₁₂ fatty acid.

18. The gastrointestinal smooth muscle contraction inhibitor according to any one of claims 13 to 17,
wherein the component (b2) is a C₆-C₁₂ fatty acid and/or a salt thereof.

19. The gastrointestinal smooth muscle contraction inhibitor according to any one of claims 13 to 18,
wherein the smooth muscle contraction inhibitor is an emulsion, and
wherein particles in the emulsion have an average particle size of less than 100 nm.

20. The gastrointestinal smooth muscle contraction inhibitor according to any one of claims 13 to 19,
which has a light transmittance of not less than 50%.

21. A method for inhibiting gastrointestinal smooth muscle contraction, the method comprising
administering to a mammal the pharmaceutical composition according to any one of claims 1 to 9.

22. Use of the pharmaceutical composition according to any one of claims 1 to 9 for inhibiting gastrointestinal smooth muscle contraction.

23. A method for inhibiting gastrointestinal smooth muscle contraction, the method comprising
administering to a mammal a pharmaceutical composition,
wherein:
the pharmaceutical composition comprises components (A), (b1), (b2), (F), and (G),
the content of component (A) is 0.01 to 5 weight %,
the content of component (b1) is 0.1 to 10 times as much by weight as that of the component (A),
the content of component (b2) is 0.1 to 10 times as much by weight as that of the component (A),
the content of component (F) is 0.1 to 10 times as much by weight as the total amount of the component (A), the component (b1), and the component (b2),
the content of component (G) is 0.01 to 2.0 weight % as benzoic acid, and
the weight ratio of the component (b2) to the component (G) [component (b2) / component (G)] is from 1/5 to 10/1, wherein the components (A), (b1), (b2), (F), and (G) are as follows:
Component (A): 1-menthol;
Component (b1): a medium-chain fatty acid triacylglycerol;
Component (b2): a medium-chain fatty acid and/or a salt thereof;
Component (F): at least one selected from the group consisting of:
a sucrose fatty acid ester;
a polyoxyethylene hydrogenated castor oil; and
a polysorbate; and
Component (G): at least one selected from the group consisting of:
benzoic acid;
p-hydroxybenzoic acid; and
a salt or an alkyl ester thereof.

24. Use of a pharmaceutical composition for inhibiting gastrointestinal smooth muscle contraction,
wherein:
the pharmaceutical composition comprises components (A), (b1), (b2), (F), and (G),
the content of component (A) is 0.01 to 5 weight %,
the content of component (b1) is 0.1 to 10 times as much by weight as that of the component (A),
the content of component (b2) is 0.1 to 10 times as much by weight as that of the component (A),
the content of component (F) is 0.1 to 10 times as much by weight as the total amount of the component (A), the component (b1), and the component (b2),
the content of component (G) is 0.01 to 2.0 weight % as benzoic acid, and
the weight ratio of the component (b2) to the component (G) [component (b2) / component (G)] is from 1/5 to 10/1, where the components (A), (b1), (b2), (F), and (G) are as follows:
Component (A): 1-menthol;
Component (b1): a medium-chain fatty acid triacylglycerol;
Component (b2): a medium-chain fatty acid and/or a salt thereof;
Component (F): at least one selected from the group consisting of:
a sucrose fatty acid ester;
a polyoxyethylene hydrogenated castor oil; and
a polysorbate; and
Component (G): at least one selected from the group consisting of:
benzoic acid;
p-hydroxybenzoic acid; and
a salt or an alkyl ester thereof.
